# EUROPÄISCHE PATENTSCHRIFT

(11) **EP 2 526 405 B1**
(45) Veröffentlichungstag und Bekanntmachung des Hinweises auf die Patenterteilung: **19.10.2016**
(21) Anmeldenummer: 11711247.4
(22) Anmeldetag: 14.01.2011
(51) Int. Cl.: G01N 21/35, G01N 21/55, G01N 33/74, G01N 33/08, G01N 21/3563, A01K 45/00, G01N 21/552, G01N 21/3577, G01N 21/359

(54) **VERFAHREN UND VORRICHTUNG ZUR BESTIMMUNG DES GESCHLECHTES VON BEFRUCHTETEN UND NICHT BEBRÜTETEN VOGELEIERN**
METHOD AND DEVICE FOR DETERMINING THE SEX OF FERTILIZED, NONINCUBATED BIRD EGGS
PROCÉDÉ ET DISPOSITIF PERMETTANT DE DÉTERMINER LE SEXE D' OEUFS D'OISEAUX FÉCONDÉS ET NON INCUBÉS

(30) Priorität: 21.01.2010 DE 102010006161
(43) Veröffentlichungstag der Anmeldung: 28.11.2012
(73) Patentinhaber: Technische Universität Dresden, 01069 Dresden (DE); Universität Leipzig, 04109 Leipzig (DE)
(72) Erfinder: STEINER, Gerald, 08340 Schwarzenberg (DE); KOCH, Edmund, 01309 Dresden (DE); KRAUTWALD-JUNGHANNS, Maria-Elisabeth, 04416 Markleeberg (DE); BARTELS, Thomas, 30989 Gehrden (DE)
(74) Vertreter: Hempel, Hartmut
(86) Internationale Anmeldenummer: PCT/DE2011/000062
(87) Internationale Veröffentlichungsnummer: WO 2011/088825

(56) Entgegenhaltungen:
- EP-A1- 1 118 267
- US-A- 5 459 316
- US-A- 5 703 366
- US-A- 6 029 080
- US-A1- 2003 172 392
- US-A1- 2008 289 578
- US-B2- 7 167 579
- GERALD STEINER ET AL: "Sexing of turkey poults by Fourier transform infrared spectroscopy", ANALYTICAL AND BIOANALYTICAL CHEMISTRY, SPRINGER, BERLIN, DE, Bd. 396, Nr. 1, 20. November 2009 (2009-11-20), Seiten 465-470, XP019777670, ISSN: 1618-2650

## Beschreibung

Die Erfindung betrifft ein Verfahren und eine Vorrichtung zur Bestimmung des Geschlechtes von befruchteten und nicht bebrüteten Vogeleiem, wobei ein Ei eine feste Eischale, ein von der Eischale und weiteren Eihüllen umgebenes Eidotter und eine dem Eidotter zugeordnete Keimscheibe aufweist und wobei eine Sonde zur Messung eines Spektrums durch ein Loch in der Eischale hindurch in Richtung zur Keimscheibe mit KeimscheibenzeHen geführt wird,
mit folgenden Schritten
- Positionierung der Sonde im Bereich der Keimscheibe,
- spektroskopische in-ovo Charakterisierung der Keimscheibenzellen,
- Erkennung des Geschlechts durch eine automatische Klassifizierung der Spektren.

Wesentlich ist dabei z.B. bei Vögeln und, da auch in wirtschaftliche Gebiete reichend, insbesondere bei Nutzgeflügel die Feststellung, ob sich z.B. aus bereits befruchteten Eiern Individuen entweder männlichen Geschlechts oder weiblichen Geschlechts entwickeln werden.

Dazu ist bereits ein Verfahren zur Geschlechtsbestimmung von Küken mittels der Infrarot-Fourier-Transform-Spektroskopie in der Druckschrift Steiner et al.: Sexing of turkey poults by fourier transform infrared spectroscopy, Analytical and Bioanalytical Chemistry, Vol. 396 (1) 2010, Seite 465-470 beschrieben, mit dem von sechs Wochen alten Küken das Geschlecht bestimmt werden kann.
Der Einsatz der Infrarotspektroskopie bei der Geschlechtsbestimmung von Jungvögeln zeigt, dass das Verfahren schnell und genau ist und von der Geflügelwirtschaft potenziell zur Identifizierung und Auswahl von Eiern mit weiblichen Embryonalstadien oder männlichen Embryonalstadien für die Brut genutzt werden kann.

Eine Vielzahl von Vogefarten, Nestlingen und insbesondere Embryonalstadien von Vögeln weisen keine äußeren Geschlechtsmerkmale auf. Für Geflügelzüchter, Tierärzte, Vogelzüchter und Ornithologen ist die exakte Geschlechtsbestimmung jedoch ein elementarer Aspekt. Doch auch für die Geflügelwirtschaft ist das Wissen von höchster Bedeutung: schnelle, objektive und kostengünstige Verfahren zu möglichst frühzeitigen Geschlechtsbestimmungen, zum Beispiel bei Hühnern und Truthühnern, sind für die Geflügelwiftschaft insofern wichtig, als dadurch das bevorzugte Geschlecht zur Ausbrütung identifiziert werden kann. Dafür wird die Fourier-Transform-Infrarot Spektroskopie eingesetzt. Das Verfahren basiert darauf, dass aus den wachsenden Konturfedern extrahierte Zellen von sechs Wochen alten männlichen und weiblichen Truthühnern verwendet werden. Die Technik nutzt die genetischen Zellinformationen und ermöglicht die direkte Geschlechtsbestimmung des Vogels.

Ein Problem besteht darin, dass der Aufwand zur geschlechtsspezifischen Bestimmung aus sehr vielen Schritten besteht und damit zeitlich sehr hoch ist.
Es ist ein Verfahren zur Bestimmung des Geschlechts von Vögeln in der Druckschrift DE 10 2007 013 107 A1 beschrieben, bei dem DNA-relevantes Zellmaterial des geschlechtlich zu bestimmenden Vogels mit Licht untersucht und die Molekülschwingungen gemessen werden, wobei das durch das Licht entstehende Spektrum der Molekülschwingungen erfasst und mit vorgegebenen sowie geschlechtsspezifische DNA-Strukturen der zu untersuchenden Vogelart repräsentierenden Referenzspektren verglichen wird und wobei aus diesem Spektralvergleich eine auf Grundlage des DNA-Gehalts des Zelimaterials basierende Geschlechtszuordnung des Vogels getroffen wird.
Die Molekülschwingungen werden dabei mittels Anwendung der Raman-Spektroskopie oder der IR-Spektroskopie gemessen, wobei z.B. das DNArelevante Zellmaterial aus dem Schaft einer jungen Feder eines Vogels entnommen werden kann. Das Zellmaterial wird auf einem Träger präpariert und mit Licht abgetastet.
In einem anderen Teilverfahren wird das Licht zur Messung der Molekülschwingungen des DNA-relevanten Zellmaterials von ungeschlüpften Vögeln durch die Eierschale hindurch auf den Embryo oder die Keimscheibe fokussiert, wobei das Spektrum der durch die Molekülschwingungen entstehenden Strahlung im Ei mit einer durch dessen Schale hindurch geführten Sonde gemessen wird.
Für die Hindurchführung der Sonde wird zur Messung des Spektrums wenigstens ein mikroskopisch kleines Loch durch die Eischale hindurch gebohrt. Das Licht wird durch den kleinen Zugang durch die Eischale unmittelbar auf die Keimscheibe als Zellmaterial fokussiert. Durch den gleichen oder einen anderen Zugang geringer Öffnungsgröße wird die Sonde eingeführt, mittels der das reflektierte und von der Sonde aufgenommene Spektrum der vorgenannten Molekülbewegung im Innern des Eis gemessen wird.

Die erhaltenen spektralen Informationen werden in einem zweiten Schritt mit Referenzdaten verglichen und einem Klassifizierungsalgorithmus zugeführt. Diese repräsentieren vorzugsweise statistisch gewonnene Daten über die zu untersuchenden Vogelspezies. Aus diesem Vergleich wird die Geschlechtszuordnung des zu untersuchenden DNA-Materials getroffen.

Ein Problem besteht darin, dass für die Einbringung einer Sonde in vorgefertigte Löcher bei einer sehr großen Anzahl von zu untersuchenden Vogeleiern ein großer zeitlicher Aufwand erforderlich ist. Außerdem muss bei einer Fokussierung des Lichts aus der Sonde auf die Keimscheibe ein erheblicher Justierungsaufwand für eine optische Abbildung betrieben werden, wobei von Ei zu Ei die Fokussierungsebene eine andere Lage aufweisen kann und damit keine Bestimmung des Geschlechts durchgeführt werden kann.

Es ist auch keine Anordnung angegeben, wie das IR-Spektrum von den Keimscheibenzellen unter in-ovo Bedingungen schnell und zuverlässig aufgenommen werden kann.

Eine Spektroskopie-Einrichtung ist in der Druckschrift US 4 835 389 A beschrieben, die ein Totalreflexions-Element enthält, das am Boden einer langen SondenRöhre angeordnet ist, die mit dem Totalreflexions-Element tief in einen Behälter mit Flüssigkeit eingetaucht wird, um mit der gesamten in der Flüssigkeit eintauchenden Oberfläche des Kristalls und vielen innerhalb des Kristalls vorgesehenen randseitlichen Totalreflexionen analytische Informationen über einen Strahlengang aufzunehmen und an die Spektroskopie-Einrichtung weiterzuleiten.

Eine Tauchsonde ist in der Druckschrift US 5 459 316 A beschrieben, die in Pulver oder Flüssigkeiten getaucht wird und mit der eine Infrarot-Spektroskopie durchgeführt wird. Die Tauchsonde besteht zumindest aus einer Röhre, die am Boden ein eingebautes, auf der Basis der abgeschwächten Totalreflexion (ATR) fungierendes ATR-Element aufweist. Das vorgesehene ATR-Element dient als Rückreflektor.

Ein refraktometrisches Verfahren zur langzeitstabilen Messung von Konzentrationen gelöster Stoffe sowie eine Vorrichtung zur Durchführung des Verfahrens sind in der Druckschrift DE 100 30 927 C1 beschrieben, wobei ein linear polarisiertes elektromagnetisches Messstrahlenbündel mittels Totalreflexion in einem Prisma geführt wird. Dabei stehen die Längsseiten und eine Schmalseite des Prismas mit der zu vermessenden Lösung im Kontakt. Im Innern des Prismas erfolgen mehrfache randseitliche Totalreflexionen des Messstrahlenbündels, das nach seinem Austritt aus dem Prisma über Phasenschieber und Analysator einem Detektor zugeleitet wird, aus dessen Signalen und einer Eichkurve die Konzentration ermittelt wird.

Eine ATR-Sonde zur Vermessung von Vollblut ist in der Druckschrift US 5 170 056 A beschrieben, wobei dabei über einen Lichtleiter Messlösungen entfernt vom Spektrometer vermessen werden können. Dabei ist die ATR-Sonde ein Teil einer Faseroptik gekoppelten Einrichtung zur Durchführung einer IR-Spektroskopie. Die die Lösung querende langzylindrische ATR-Sonde weist an den beiden Stirnseiten angekoppelte Anschlüsse der Lichtleiter auf, über die die InfrarotStrahlung durch die ATR-Sonde geführt wird. Aus den vielen abgeschwächten randseitlichen Totalreflexionen innerhalb der ATR-Sonde beim Durchgang der Strahlenbündel durch die ATR-Sonde hindurch wird die detektierte IR-Strahlung gemessen und zur Parameterbestimmung des Blutes genutzt.

Probleme bestehen darin, dass mit den Ausbildungen der genannten Sonden mit den vielen randseitlichen Totalreflexionen Verfälschungen in den detektierten Spektren auftreten können und mit den detektierten Totalreflexionsbündein auch keine präzise Positionierung der Kristallspitze auf Keimscheibenzellen möglich ist. Somit kann keine präzise Bestimmung des Geschlechts von Vogeleiern durchgeführt werden.

Ein Verfahren zur Lokalisierung von Keimscheiben in Vogeleiern ist in dem Dokument US 2008/0289578 A1 mit folgenden Schritten beschrieben:
- Bildung einer Öffnung in der Schale des Eis an einer Stelle, unter der die Keimscheibe unter Berücksichtigung der Ausrichtung des Eis positioniert ist, und Freilegung der inneren Schalenmembran,
- Einbringen eines flüssigen Materials in den Bereich der inneren Schalenmembran zur Verbesserung der Transparenz der Schalenmembran,
- Aufnahme eines Bildes von der freiliegenden inneren Schalenmembran und des darunter liegenden Materials,
- Verarbeitung des erfassten Bildes zur Verbesserung der Erkennbarkeit/Sichtbarkeit der Keimscheibe in dem Bild,
- Bestimmung der Lokalisierungs-Koordinaten der Keimscheibe innerhalb des Bildes,
- Übermittlung der Lokalisierungs-Koordinaten an eine Ei-Verarbeitungsvorrichtung.
Es soll die Erkennbarkeit der Keimscheibe verbessert werden.

In dem Verfahren zur nicht-invasiven Lokalisierung der Keimscheibe von Vogeleiern wird jedoch kein Anspruch auf die Geschlechtsbestimmung des unbebrüteten Eis erhoben. Es wird die Möglichkeit einer Geschlechtsbestimmung eines Embryos, also eines bebrüteten Eis erwähnt. Die Bebrütung des Eis ist erforderlich, um ein größere Keimscheibe zu erhalten und damit die Erkennbarkeit zu verbessern. Zusätzlich wird zur Erkennungsverbesserung flüssiges Material unter die Eischale eingebracht.
Die in dem Verfahren genannte Erkennung des Geschlechts beruht nicht auf molekularen und damit objektiven Informationen, sondern lediglich auf morphologischen Merkmalen. Eine oberhalb des geöffneten Eis angebrachte Kamera kann den Embryo erfassen. Aus der Größe und Form des Embryos lassen sich Rückschlüsse auf das Geschlecht ziehen. Allerdings sind keine weiteren Details genannt oder beschrieben, wie das erreicht werden kann.

Aus dem Dokument US 2008/0289578 A1 kann nicht abgeleitet werden, wie eine Geschlechtsbestimmung des befruchteten, aber unbebrüteten Eis erfolgen kann. Es werden zudem keine Verfahren genannt, wie molekulare Informationen zur Größe des Genoms, d.h. zur Anzahl der Basensequenzen, gewonnenen und ausgewertet werden müssen.

Im Gegenteil: Zur Verbesserung des zwischen Keimscheibe und Eidotter herauszuarbeitenden Kontrastes, der für eine verbesserte Erkennung der Keimscheibe gemäß Aufgabenstellung dem Dokument US 2008/0289578 A1 notwendig ist, wird flüssiges Material in den Bereich der inneren Schalenmembran der geöffneten Eischale eingebracht. Das stellt aber einen hohen Bearbeitungsaufwand dar.
Eine Tauchsonde für Proben aus Pulver und aus Flüssigkeiten wird in dem Dokument US 5 459 316 beschrieben, wobei mit der Tauchsonde eine InfrarotSpektroskopie durchgeführt wird. Es wird ein ATR-Element eingesetzt, das an der Unterseite eines Rohres angeordnet ist und in dem die Strahlung verläuft. Das ATR-Element besitzt eine Spitze, die entweder konisch oder dachförmig ausgebildet ist und in die Probe eingeführt wird.
Die ins ATR-Element eintretende Strahlung wird zweimal an den AbtastEingriffsflächen reflektiert, wobei dabei eine Abschwächung der reflektierten Strahlung durch das Probenmaterial auftritt. Das ATR-Element kann auch als Retroreflektor arbeiten, wodurch die austretende Strahlung in einem Pfad parallel zur eintretenden Strahlung folgt.

Dabei wird ein spezieller länglicher Kristall mit Spitze in das zu untersuchende Pulver oder in die zu untersuchende Flüssigkeit eingesetzt. Zudem wird beschrieben, wie das Licht zum Kristall geführt wird und wie die gewonnenen spektralen Signale einem Detektor zugeführt werden können. Es wird aber keine Lösung für die Messung der Keimscheibe eines Eis angegeben. Insbesondere wird kein Verfahren dargelegt, wie der Kristall exakt in der Keimscheibe positioniert werden kann. Ebenso wenig sind keine Verfahren zur Geschlechtsbestimmung beschrieben.

Da weder das Dokument US 2008/0289578 A1 noch das Dokument US 5 459 316 ein Verfahren und eine Vorrichtung zur Bestimmung des Geschlechtes von befruchteten und nicht bebrüteten Vogeleiem enthalten, wird eingeschätzt, dass das Dokument US 2008/0289578 A1 und das Dokument US 5 459 316 in zwei verschieden technische Kategorien einzuordnende Lösungen darstellen, die aber nicht in ihrer Kombination zur Geschlechtsbestimmung am nicht bebrüteten Vogelei führen.

Ein Verfahren zur Überprüfung des Vorhandenseins eines lebenden Embryos in einem bebrüteten Geflügelei ist in dem Dokument EP 1 118 287 A1 beschrieben.
Dabei werden zwei Elektroden am Ei angebracht, um einen leitenden Kontakt mit der äußeren Oberfläche der Eischale zu erreichen.
Es erfolgt eine Verstärkung jedes mittels der Elektroden erfassten analogen Signals.
Nach der Verstärkung der erfassten Signale wird mit einem Programm in einem Computer festgestellt, ob die erfassten Signale von einer Herzaktivität des Embryos stammen.
Die erhaltenen Informationen sollen die Anwesenheit eines lebenden Embryos im Ei bestätigen oder nicht bestätigen.

Eine Positionierung eines Kristalls vor einer Keimscheibe einschließlich der Geschlechtsbestimmung aus der Keimscheibe wird nicht beschrieben.

Ein nicht-invasives Verfahren und eine Vorrichtung zur Bestimmung sind in dem Dokument US 6 029 080 A beschrieben, ob befruchtete lebende Eier einen Embryo mit männlichen Geschlechtsmerkmalen oder weiblichen Geschlechtsmerkmalen enthalten, wobei das Verfahren folgende Schritte aufweist
- Bereitstellung einer Vielzahl von Eiern,
- Bestrahlung der Geschlechtsorgane des Embryos jedes der vielen Eier mit Strahlenbündeln durch Bewegung der Vorrichtung, die die Strahlung oder die Eier ohne spürbare Krafteinwirkung auf den Embryo, um ein erfassbares Signal von den Geschlechtsorganen des Embryos zu erhalten, einstellen,
- Bestimmung des Geschlechtes des Embryos von jedem der Eier mittels einer Analyse des Signals.
Die Bestrahlung wird mittels des NMR-Verfahrens durchgeführt.

Auch in dem Dokument US 6 029 080 A erfolgt eine Bestimmung der Geschlechtsmerkmale aus bebrüteten Eiern, insbesondere aus den Embryos. Auch hier sind vor der Geschlechtsbestimmung ein Energieaufwand und ein Zeitaufwand vorhanden, um die Keimscheibe zu vergrößern, was aber vermieden werden soll.

Mit dem Verfahren und der Vorrichtung zur Geschlechtsbestimmung im Dokument US 7 107 579 B2 soll eine visuelle Bestimmung vermieden werden.
In dem Dokument US 7 107 579 B2 wird das Geschlecht des befruchteten Eis unter Verwendung von Parametern, die Merkmale der Oberfläche des Eis darstellen, bestimmt, wobei die Bilddaten von der Kontur der befruchteten Eizelle durch Aufnehmen eines Bildes des oberen Teils des befruchteten Eis mittels mindestens zweier Strahlenbündel ermittelt werden und wobei das Ei auf einer Aufsetzeinrichtung ruht.
In dem Dokument US 7 107 579 B2 wird keine Keimscheibenermittlung, sondern eine Bestimmung des Geschlechtes aus der Konturausbildung der Schale eines befruchteten Eis durchgeführt.

Ein Verfahren zum Einbringen einer Öffnung in ein Vogelei ist in dem Dokument US 2003/172392 A1 beschrieben, wobei das Ei einen Embryo (auch eine Keimscheibe) enthält,
bestehend aus folgenden Schritten:
- Ausrichten des Vogeleis in eine vorbestimmte Position,
- Einfügen einer kleinen Öffnung in die Ei-Oberfläche,
- Einbringen einer Detektor-Vorrichtung durch die kleine Öffnung in die Ei-Oberfläche,
- Durchstechen der inneren Oberflächen-Membran mit der Detektor-Vorrichtung, wobei die innere Oberflächen-Membran im Wesentlichen vor dem Eindringen der Detektor-Vorrichtung dadurch unbeschädigt bleibt,
- Zurückziehen der Vorrichtung aus dem Ei.

In dem Dokument US 2003/172392 A1 wird die Ausbildung der Position einer Detektor-Vorrichtung innerhalb der Ei-Oberfläche, wobei mittels der Detektor-Vorrichtung Bilder des Embryos aufgenommen werden, beschrieben. Außerdem sollen in das Ei über die Öffnung Stoffe in die Nähe des Embryos gebracht werden. Es ist keine Bestimmung des Geschlechtes eines befruchteten und unbebrüteten Vogeleis beschrieben.

Alle anderen mit Embryonen versehenen Eier aus den meinsten vorstehend genannten Dokumenten brauchen einen Energie- und Zeitaufwand, bis eine größere Keimscheibe erreicht wird, um eine relativ genaue Geschlechtsbestimmung durchführen zu können. Gerade dieser Aufwand soll vermieden werden.

Eine Kristall-Anordnung zur optischen Analyse von Proben ist in dem Dokument US 5 703 366 A beschrieben, wobei die Kristallanordnung zumindest aus einem ersten Kristall-Körper und einem zweiten Kristall-Körper besteht.
Die beiden Kristall-Körper haben im Wesentlichen einen gleichen Brechungsindex und stehen mit einer optischen Schnittstelle in Verbindung. Diese Schnittstelle kann durch die Kristallflächen in innigem Kontakt stehen oder auch mit einem dritten Kristall-Körper kombiniert sein.
Die fokussierte Infrarotenergie, die von dem ersten Kristall-Körper übertragen wird, wird intern innerhalb des zweiten Kristall-Körpers reflektiert, um eine bestimmte Kodierung der Infrarotenergie zur Probe zu erhalten. Die kodierte Infrarotenergie wird zurück in den ersten Kristall-Körper zur Übertragung zu einem Detektor reflektiert.

Die in dem Dokument US 5 703 366 A enthaltene Kristall-Anordnung wird nicht zu einer Geschlechtsbestimmung von befruchteten und unbebrüteten Vogeleiern eingesetzt.
Der Erfindung liegt deshalb die Aufgabe zugrunde, ein Verfahren und eine Vorrichtung zur Bestimmung des Geschlechtes von befruchteten und nicht bebrüteten Vogeleiern anzugeben, wobei die Möglichkeit zur Positionierung der Keimscheibe für eine präzise Bestimmung des Geschlechtes eines Vogeleis verbessert werden soll. Außerdem soH damit auch der Energie- und Zeitaufwand gegen über den Geschlechtsbestimmungen von bebrüteten Vogeleiern verringert werden.

Die Aufgabe wird durch ein Verfahren mit den Merkmalen des Patentanspruchs 1 und durch eine Vorrichtung mit den Merkmalen des Patentanspruchs 7 gelöst.
Das Verfahren zur Bestimmung des Geschlechts von befruchteten und nicht bebrüteten Vogeleiern, wobei ein Ei eine feste Eischale, und ein von der Eischale und weiteren Eihüllen umgebenes Eidotter und eine dem Eidotter zugeordnete Keimscheibe aufweist, wobei eine Sonde zur Messung eines Spektrums durch ein Loch in der Eischale hindurch in Richtung zur Keimscheibe mit Keimscheibenzellen geführt wird,
weist gemäß dem Patentanspruch 1
folgende Schritte auf:
- Positionierung der Sonde im Bereich der Keimscheibe,
- spektroskopische in-ovo Charakterisierung der Keimscheibenzellen,
- Erkennung des Geschlechts durch eine automatische Klassifizierung von Spektren,
wobei als Sonde ein optischer Kristall eingesetzt wird, mit dem eine schnelle und rückwirkungsfreie Aufnahme eines Infrarot- und/oder Nahinfrarotspektrums bei Nutzung einer abgeschwächten Totalreflexion innerhalb des optischen Kristalls durch ein evaneszentes Feld im Bereich der Keimscheibe durchgeführt wird, wobei die Extinktion infolge einer spektralen Absorption von geschlechtsspezifischen Keimscheibenzellen erfolgt, wobei die Positionierung des optischen Kristalls durch eine permanente automatische Auswertung der rückgeführten Spektren bis zur Bestimmung der geschlechtsspezifischen Keimscheibenzellen begleitet wird, bis das Geschlecht des befruchteten Eis von einer Auswerteeinheit ausgewertet und von einer Anzeigeeinheit eindeutig angezeigt wird,
wobei die automatische Positionierung des optischen Kristalls in Bezug auf die Keimscheibenzellen mit einer Kristallspitze oder Ausgangsfläche solange durchgeführt wird, bis das evaneszente Feld der Totalreflexion an der Kristallspitze oder Ausgangsfläche die Keimscheibe erfasst und der Kristall in eine finale Scheibenzuordnungsposition gebracht ist, bei der das evaneszente Feld in Wechselwirkung mit den Keimscheibenzellen tritt und
wobei während des Positionierungsvorgangs permanent rückgeführte IR- und/ oder NIR-Spektren aufgezeichnet und einer Auswertung zugeführt werden, wobei eine automatische Klassifizierung der totalreflektierten Spektren anhand des spektralen Fingerabdruckes in Proteine, Lipiden und Nucleinsäuren erfolgt.

Die Scheibenzuordnungsposition des optischen Kristalls kann in alle Raumrichtungen gerichtet sein, um die Keimscheibe präzise zu treffen.

Bei der geschlechtsspezfischen Absorption des einfallenden IR- und/oder NIR-Lichts können die Keimscheibenzellen anhand von Absorptionsbanden der Nukleinsäuren (DNA und RNA) derart identifiziert werden, dass das Geschlecht des geprüften Eis bestimmt und angezeigt wird.

Die Auswertung in der Auswerteeinheit kann mittels der IR- und/oder NIR-Spektroskopie oder der Fourier-Transform-Infrarot-Spektroskopie durchgeführt werden.

Es können gleichzeitig mehrere Eier in einer Feldanordnung und voneinander beabstandet vermessen werden, wobei entweder feldartig voneinander beabstandet mehrere optische Kristalle angeordnet werden, die jeweils mit einer separaten optischen Faser gekoppelt sind oder wobei das Licht mittels Spiegel oder Spiegelsystemen zur Strahlablenkung von der spektralen Lichtquelle aus und nach geschlechtsspezifischer Absorption auf einen IR- und/oder NIR-empfindlichen Detektor mit feldartig voneinander beabstandeten Detektorelementen oder Pixeln geleitet wird.

Die Vorrichtung zur Bestimmung des Geschlechtes von befruchteten und nicht bebrüteten Vogeleiem, wobei das Ei eine feste Eischale, ein von der Eischale und weiteren Eihüllen umgebenes Eidotter und eine dem Eidotter zugeordnete Keimscheibe aufweist, wobei eine Sonde zur Messung eines Spektrums durch ein Loch der Eischale hindurch in Richtung zur Keimscheibe mit Keimscheibenzellen geführt ist, nach dem vorgenannten Verfahren,
enthält gemäß dem Patentanspruch 7
- mindestens eine Eipositions-Auflage zur Arretierung mindestens eines Eis,
- mindestens eine Höhenverstelleinrichtung mit mindestens einem Haltearm ,
- mindestens einen als Sonde ausgebildeten optischen Kristall, der an dem Haltearm befestigt ist,
- mindestens eine Steuereinheit zumindest zum Betreiben der eiarretierenden Eipositions-Auflage und der Höhenverstelleinnchtung,
- mindestens eine spektrale Lichtquelle, die einen IR- und/oder NIR-Lichtstrahl aussendet,
- mindestens einen Detektor zur Aufnahme des rückgeführten IR- und/oder NIR-Lichts,
- mindestens ein optisches Element für einen geführten Strahlengang zwischen der spektralen Lichtquelle und dem optischen Kristall sowie für einen rückgeführten Strahlengang zwischen dem optischen Kristall und dem Detektor sowie
- eine mit dem Detektor verbundene Auswerteeinheit und eine Anzeigeeinheit,
wobei mit der Höhenverstelleinrichtung die Höhe des Haltearms und somit des optischen Kristalls in Bezug auf die Lage der Keimscheibe eingestellt wird und der optische Kristall im Bereich der Keimscheibe in einer Scheibenzuordnungsposition positioniert ist, in der über den optischen Kristall ein sich bei Totalreflexion an der zur Keimscheibe gerichteten Ausgangsfläche ausbildendes evaneszentes Feld auf die Keimscheibe übergreift und die darin befindlichen Keimscheibenzellen wechselwirkend mit dem evaneszenten Feld eine geschlechtsspezifische Absorption des Lichts aus dem eingeführten Strahlengang vornehmen, wobei das an der Ausgangsfläche totalreflektierte Licht über den rückgeführten Strahlengang innerhalb des Kristalls und schließlich über das optische Element zur Registrierung zum Detektor geführt ist, von dem aus die registrierten spektralen Signale zur Auswertung und Anzeige des Geschlechts übermittelt werden,
wobei eine Einrichtung zur Feststellung der Lage der Keimscheibe, die Höhenverstelleinrichtung und die Eipositions-Auflage in der Steuereinheit mittels programmtechnischer Mittel zu einer Koordinierung der Lage der Keimscheibe und der Scheibenzuordnungsposition des optischen Kristalls verbunden sind, womit eine präzise Scheibenzuordnungsposition des optischen Kristalls im Bereich der Keimscheibe festgelegt ist.

Das Loch in der Eischale kann entweder vor der Einbringung des optischen Kristalls in die Scheibenzuordnungsposition bereits vorhanden sein oder während des Durchtritts des optischen Kristalls durch die Eischale vom Kristall selbst eingebracht sein.

Mit der Einrichtung zur Feststellung der Lage der Keimscheibe innerhalb des Eis kann über den Haltearm, der höhenverstellbar und verschwenkbar ausgebildet ist, die Scheibenzuordnungsposition des optischen Kristalls im Bereich der Keimscheibe eingestellt werden.

Die Einrichtung zur Feststellung der Lage der Keimscheibe kann über mindestens eine versorgungs- und signaltechnische Leitung mit der Steuereinheit verbunden sein.

Das optische Element kann eine flexible optische Faser sein und der optische Kristall mit der flexiblen optischen Faser verbunden sein, wobei der Lichtstrahl sowohl von der spektralen Lichtquelle zum optischen Kristall als auch vom optischen Kristall zum Detektor durch die Faser geleitet wird.

Der optische Kristall kann mit verschieden ausgebildeten Kristallendflächen oder Kristallenden, mit einer ebenen, einer runden oder ovalen Ausgangsfläche oder aber auch mit einer spitzen Kristallspitze versehen sein.

Der optische Kristall kann zylindrisch ausgebildet sein und sich in einem Metallmantel gehaltert befinden, wobei der Metallmantel mit dem Haltearm in Verbindung steht.

Zwischen dem Metallmantel und der Oberfläche des optischen Kristalls kann zur Verbesserung der Reflexion an der Oberfläche eine Goldschicht angebracht sein.

Der optische Kristall kann nadelförmig im Bereich der unteren Ausgangsfläche ausgebildet sein.

Der optische Kristall kann aus IR-transparentem und zugleich hartem Material aus Germanium, Silizium, Kalziumfluorid (CaF₂), Bariumfluorid (BaF₂), Zinkselenid (ZnSe) oder Zinksulfid (ZnS) bestehen.

Der optische Kristall kann für Messungen mit NIR-Licht aus Glas oder Saphir bestehen.

Der optische Kristall kann aus einem Komposit bestehen, wobei bei der Nadelförmigkeit des Kristalls die Kristallspitze im Bereich der Ausgangsfläche aus Diamant besteht.

Als IR-und/oder NIR-sensitiver Detektor kann ein pixellierter Detektor eingesetzt sein, um gleichzeitig und parallel rückgeführte Spektren von vielen zu prüfenden Eiern aus den zugeordneten optischen Kristallen pixelzugeordnet zu erfassen.

Die Vorteile bestehen darin, dass bei der Durchführung des Verfahrens dem Ei kein Material entnommen werden muss. Es werden auch keine Fremdstoffe zur Signalerfassung in das Ei eingebracht. Zudem erfolgt kein Eintrag von Energie, zum Beispiel in Form von Wärme oder Licht.

Das erfindungsgemäße Verfahren ist ein Verfahren, das für die Geflügelwirtschaft bei der Auswahl von Eiern mit weiblichen Embryonalstadien oder männlichen Embryonalstadien für die Zucht, Eiproduktion oder Mast genau genug ist, wodurch die Tötung von Millionen Küken des unerwünschten Geschlechts kurz nach dem Schlüpfen vermieden werden kann.

Der optische Kristall kann in seiner Scheibenzuordnungsposition mit seiner unteren Ausgangsfläche auf die Keimscheibe gerichtet sein, wobei sich die Keimscheibe zumindest in einem Teil des evaneszenten Feldes zur Absorption des IR-und/oder NIR-Lichtes befindet.

Der optische Kristall kann so ausgelegt sein, dass er den IR- und/oder NIR-Lichtstrahl führt und an der Spitze im Bereich der Ausgangsfläche eine Totalreflexion auftritt.

Als spektrale Lichtquellen für IR- und/oder NIR-Licht können herkömmliche Spektrometer mit breitbandigen thermischen Quellen, Laserdioden oder Lichtquellen mit Filtern, insbesondere Interferenzfiltern eingesetzt werden.

Der Vorteil der erfindungsgemäßen Vorrichtung ist die Möglichkeit der Paraltelisierung der Messung an vielen Vogeleiem und damit eine schnelle gleichzeitige geschlechtsspezifische Bestimmung der Vogeleier.

Weiterbildungen und weitere Ausgestaltungen des Verfahrens und der Vorrichtung werden in weiteren Unteransprüchen angegeben.

Die Erfindung wird mittels mehreren Ausführungsbeispielen anhand mehrerer Zeichnungen näher erläutert.

Es zeigt:
- Fig. 1: eine schematische Darstellung der Vorrichtung zur Bestimmung des Geschlechtes von befruchteten und nicht bebrüteten Vogeleiern,
- Fig. 2: eine vergrößerte Darstellung des optischen Kristalls während einer spektrometrischen Messung von Keimscheibenzellen eines Vogeleis in einer Scheibenzuordnungsposition,
- Fig. 3: eine vergrößerte Darstellung des optischen Kristalls mit einer Spitze zum Durchstoßen der Eischale und Ausbildung eines Loches sowie mit Totalreflexion und Mantelfläche,
- Fig. 4a: Fourier-Transform-Infrarot-(FTIR)-Spektren von Keimscheibenzellen männlicher Vogeleiern und weiblicher Vogeleier, wobei die Pfeile spektrale Bereiche zur geschlechtsspezifischen Charakterisierung/Unterscheidung darstellen,

- Fig. 4b: vergrößerter Ausschnitt der auszuwertenden (FTIR)-Spektren nach Fig. 4a im Bereich der Pfeile,
- Fig. 5: eine schematische Darstellung einer Vorrichtung zur Bestimmung des Geschlechtes von befruchteten und nicht bebrüteten Vogeleiern mittels einer parallelen Messung von vielen Vogeleiern unter Einsatz von optischen Fasern, die endseitig zum Ei gerichtet mit optischen Kristallen verbunden sind, zur Strahlführung,
- Fig. 6: eine schematische Darstellung einer Vorrichtung zur Bestimmung des Geschlechtes von befruchteten und nicht bebrüteten Vogeleiern mittels einer parallelen Messung von vielen Vogeleiern nach Fig. 5 unter Einsatz von Spiegeln/Spiegelsystemen zur Strahlführung und lichteinfaliswinkelspezifisch angeschliffenen Kristalleingangsflächen anstelle der optischen Fasern und
- Fig. 7: ein Blockschema zur Erläuterung der Funktionsweise zur Klassifizierung der rückgeführten und detektierten Spektren.

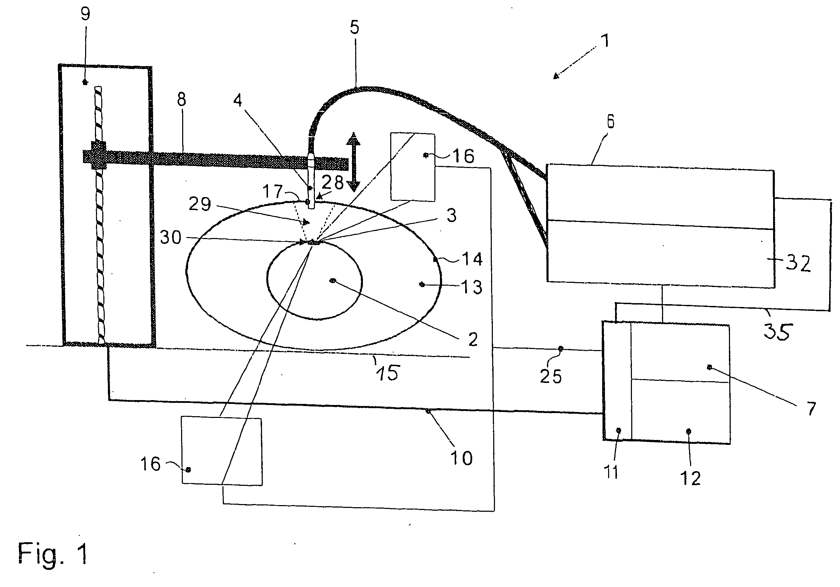

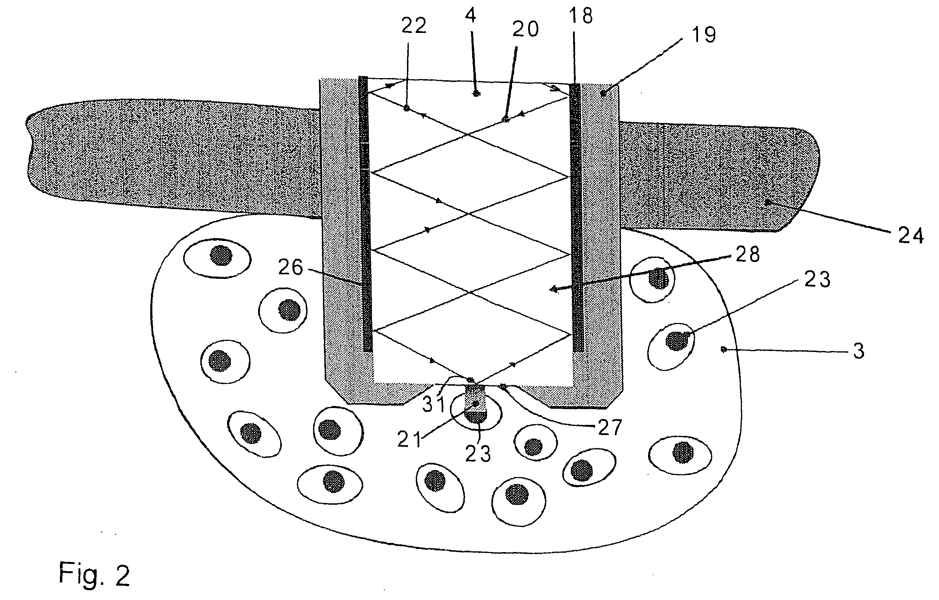

Im Folgenden werden die beiden Fig. 1 und Fig. 2 gemeinsam betrachtet.
In Fig. 1 ist in einer schematischen Darstellung eine Vorrichtung 1 zur Bestimmung des Geschlechtes eines befruchteten und nicht bebrüteten Vogeleis 13 gezeigt, wobei das Vogelei 13 eine Eischale 14, ein von der Eischale und weiteren Eihüllen umgebenes Eidotter 2 und eine dem Eidotter 2 zugeordnete Keimscheibe 3 aufweist und wobei eine Sonde 4 zur Messung eines Spektrums durch ein mikroskopisch kleines Loch 17 der Eischale 14 hindurch in Richtung zur Keimscheibe 3 mit Keimscheibenzellen 23 geführt ist, wobei die Vorrichtung 1 folgende Bauteile/Baugruppen enthält:
- eine Eipositions-Auflage 15 mit einer Arretierung des Vogeleis 13,
- eine Höhenverstelleinrichtung 9 mit einem Haltearm 8,
- einen als Sonde 4 ausgebildeten optischen Kristall, der an dem Haltearm 8 befestigt ist,
- mindestens ein optisches Element 5 zur geführten Strahlführung und rückgeführten Strahlführung,
- eine spektrale Lichtquelle 6 für IR- und/oder NIR-Licht, die über dem optischen Element 5 mit dem optischen Kristall 4 in Verbindung steht und die Licht des vorgegebenen Infrarot- und/oder Nahinfrarot-Wellenlängenbereiches aussendet,
- einen Detektor 32, der das über das optische Element 5 rückgeführte Licht empfängt,
- mindestens eine Steuereinheit 11 zumindest für die eiarretierende Eipositions-Auflage 15 und für die Höhenverstellleinrichtung 9 sowie
- eine mit dem Detektor 32 verbundene Auswerteeinheit 7 und eine Anzeigeeinheit 12,
wobei mit der Höhenverstelleinrichtung 9 die Höhe des Haltearms 8 und somit des optischen Kristalls 4 in Bezug auf den Ort der oberhalb des Eidotters 2 angeordneten Keimscheibe 3 einstellbar ist und der optische Kristall 4 im Bereich 29 oberhalb der Keimscheibe 3 in einer Scheibenzuordnungsposition 28 positionierbar ist, in der über den optischen Kristall 4 ein in Fig. 2 gezeigtes, sich bei Totalreflexion 31 an der unteren, zur Keimscheibe 3 gerichteten Ausgangsfläche 27 ausbildendes evaneszentes Feld 21 auf die Keimscheibe 3 übergreift und die darin befindlichen Keimscheibenzellen 23 wechselwirkend mit dem evaneszenten Feld 21 eine geschlechtsspezifische Absorption des Lichts aus dem einfallenden Strahlengang 20 vornehmen, wobei das an der unteren Ausgangsfläche 27 totalreflektierte Licht über die rückgeführte Strahlführung 22 innerhalb des optischen Kristalls 4 und schließlich des optischen Elements 5 zum Detektor 32 geführt ist, so dass über den optischen Kristall 4 unterschiedlich abgeschwächtes totalreflektiertes Licht zum Detektor 32 zur klassifizierenden Auswertung und Anzeige der geschlechtsspezifischen Absorption übermittelt wird.

Als spektrale Lichtquelle 6 für IR- und/oder NIR-Licht kann in Fig. 1 ein Spektrometer eingesetzt sein.

Die in Fig. 1 gezeigte Scheibenzuordnungsposition 28 des optischen Kristalls 4 ist im Bereich 29 oberhalb der Keimscheibe 3 durch das in der Eischale 14 eingebrachte Loch 17 vorgesehen, wobei das Loch 17 in der Eischale 14 entweder vor der Einbringung des optischen Kristalls 4 auf die Scheibenzuordnungsposition 28 bereits vorhanden ist oder während des Durchtritts des optischen Kristalls 4 durch die Eischale 14 hindurch vom optischen Kristall 4 selbst vorgenommen wird.

Der erfindungsgemäßen Vorrichtung 1 in Fig. 1 kann vorzugsweise eine Einrichtung 16 zur Feststellung der Lage 30 der Keimscheibe 3 im Vogelei 13 zugeordnet sein.

Mit der Einrichtung 16 zur Feststellung der Lage 30 der Keimscheibe 3 im Vogelei 13 kann über den Haltearm 8, der höhenverstellbar und verschwenkbar ausgebildet sein kann, die Position, insbesondere die Scheibenzuordnungsposition 28 des optischen Kristalls 4 in dem Vogelei 13 weitgehend in einem Bereich 29 oberhalb und vorzugsweise senkrecht zur Keimscheibe 3 eingestellt werden.

Der Haltearm 8 kann aber auch gesteuert über eine zur Höhenverstelleinrichtung 9 geführte Steuerleitung 10 zur Lochinstallierung in der Eischale 14 mit stoßartig oder schlagartig ausführbaren mechanischen Impulsen, gegebenenfalls begleitet von einer kurzen Drehung, beaufschlagbar sein.

Die Einrichtung 16 zur Feststellung der Lage 30 der Keimscheibe 3 in dem Vogelei 13 kann über mindestens eine energieversorgungs- und signaltechnische Leitung 25 auch mit der Steuereinheit 11 verbunden sein, mit der auch die spektrale Lichtquelle 6 für IR- und/oder NIR-Licht energieversorgungs- und signaltechnisch über die Leitung 35 in Verbindung steht.

Dabei kann die Einrichtung 16 zur Feststellung der Lage 30 der Keimscheibe 3 in dem Vogelei 13 mit der Höhenverstelleinrichtung 9 und der Eipositions-Auflage 15 über die Steuerleitung 10 in Verbindung stehen.

Die Einrichtung 16 zur Feststellung der Lage 30 der Keimscheibe 3, die Höhenverstelleinrichtung 9 und die Eipositions-Auflage 15 können in der Steuereinheit 11 mittels programmtechnischer Mittel zu einer gemeinsamen Koordinierung verbunden sein, mit der die präzise Scheibenzuordnungsposition 28 des optischen Kristalls 4 oberhalb der Keimscheibe 3 des Vogeleis 13 festgelegt werden kann.

Der zweckmäßige Vorgang des Messens bei Vogeleiern 13 besteht darin, dass das Eidotter 2 sich durch die vorhandenen Hagelschnüre (Chalazen) derart verdrehen kann, dass sich die Keimscheibe 3 im oberen Teil des Eidotters 2 befindet.
Es ist zweckmäßig, die Lage 30 der Keimscheibe 3 vor der Bestimmung des Geschlechts mittels der Einrichtung 16 zur Feststellung der Lage 30 der Keimscheibe 3 zu bestimmen. Dann erst kann durch die steuerbare Höhenverstelleinrichtung 9 eine Lochinstallation vorgenommen und eine Scheibenzuordnungsposition 28 des optischen Kristalls 4 gegenüber der Keimscheibe 3 herbeigeführt werden.

In Fig. 2 ist eine vergrößerte Darstellung des optischen Kristalls 4 während der spektrometrischen Messung von Absorptionsspektren der Keimscheibenzellen 23 auf der Keimscheibe 3 eines Vogeleis 13 gezeigt, wobei der optische Kristall 4 zylindrisch ausgebildet ist und sich in einem Metallmantel 19 gehaltert befindet. Der Metallmantel 19 steht, wie in Fig. 1 gezeigt ist, mit dem Haltearm 8 in Verbindung. Zwischen dem Metallmantel 19 und der Oberfläche 26 des optischen Kristalls 4 kann zur Verbesserung der Reflexion bzw. Totalreflexion 31 an der Oberfläche 26 des Kristalls 4 eine dünne Goldschicht 18 angebracht sein.

Der mantelgefasste optische Kristall 4 ist in Fig. 2 in seiner Scheibenzuordnungsposition 28 mit seiner unteren mantelfreien Ausgangsfläche 27 auf die Keimscheibe 3 gerichtet. Vorzugsweise kann die Scheibenzuordnungsposition 28 des optischen Kristalls 4 durch die Dottermembran 24 hindurch bis unmittelbar über der Keimscheibe 3 zur Feldübergreifung des evaneszenten Feldes 21 auf die Keimscheibenzellen 23 festgelegt werden.

Durch den einfallenden Strahlengang 20 im Rahmen der Strahlführung wird das IR- und/oder NIR-Licht aus dem Spektrometer 6 heraus zu der unteren, der Keimscheibe 3 gegenüber positionierten Ausgangsfläche 27 des optischen Kristalls 4 geführt, wobei sich dort durch die strahleingestellte Totalreflexion 31 das evaneszente Feld 21 ausbildet, das je nach Absorption des IR- und/oder NIR-Lichts an den Keimscheibenzellen 23 den an der Ausgangsfläche 27 kristallinnenseitig totalreflektierenden Strahlengang 22 durch Absorption spektral ändert, wobei das abgeschwächte IR- und/oder NIR-Licht des Strahlengangs 22 durch den optischen Kristall 4 hindurch über das optische Element 5 zur Strahlführung dem Detektor 32 nachfolgend der Auswerteeinheit 7 zur spektralen Auswertung zugeführt wird.

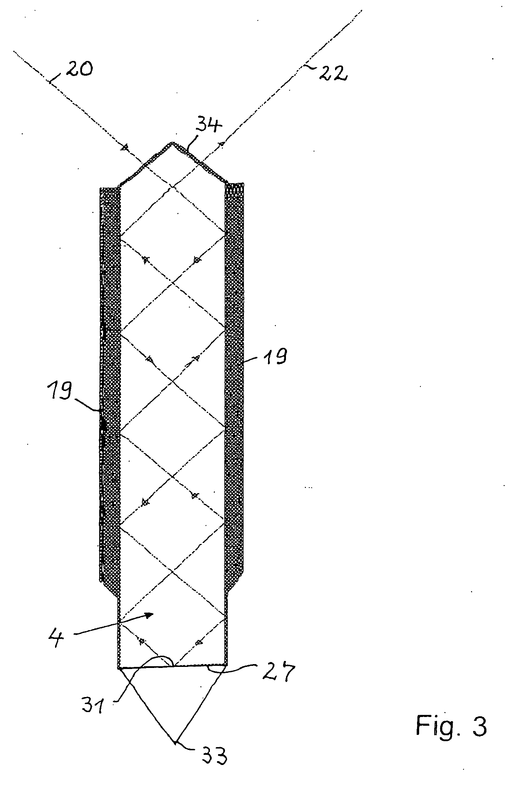

In Fig. 3 ist der optische Kristall 4 als zylindrischer Körper ausgebildet, der an seiner unteren, zur Keimscheibe 3 gerichteten Ausgangsfläche 27 zusätzlich eine Kristallspitze 33 aufweist. Die Kristallspitze 33 in Fig. 3 ist kegelförmig ausgebildet und dient auch zur Einbringung des Lochs 17 durch die Eischale 14. Der optische Kristall 4 besitzt eine auf der der Ausgangsfläche 27 oder der Kegelspitze 33 entgegengesetzte Kegelfläche 34, durch die der geführte Strahlengang 20 und der rückgeführte Strahlengang 22 führen.
Die Kristallspitze 33 des optischen Kristalls 4 kann z.B. aber auch nadelförmig ausgebildet sein.

Ein wesentlicher Punkt während der Durchführung des Verfahrens ist die Reinigung/Sterilisierung der Kristallspitze 33 und/oder der Ausgangsfläche 27. Für unverfälschte Aufnahmen der Spektren dürfen weder Fremdstoffe noch Keime in die nachfolgenden Vogeleier 13 eingebracht werden. Deshalb kann der erfindungsgemäßen Vorrichtung 1 zur Bestimmung des Geschlechtes eines befruchteten und nicht bebrüteten Vogeleis 13 eine Einrichtung zur Reinigung und Sterilisierung (nicht eingezeichnet) zugeordnet sein, die die Kristallspitze 33 nach jeder Messung reinigt und sterilisiert. Die Einrichtung zur Reinigung und Sterilisierung kann auf der Basis einer Dampfsterifisierung und/oder mittels chemischer Mittel durchgeführt werden. Es dürfen auch keine Flüssigkeiten in den Zwischenraum zwischen dem optischen Kristall 4 und dem Metallmantel 19 bzw. der reflektierenden Goldschicht 18 eindringen. Die Einrichtung zur Reinigung und Sterilisierung kann an der Höhenverstelleinrichtung 9 ebenso wie der Haltearm 8 gehaltert sein und je nach Beendigung des Messvorgangs zur Reinigung und Sterilisierung des optischen Kristalls 4 bzw. der Kristallspitze 33 aktiviert werden.

Die nadelförmige oder kegelförmige Kristallspitze 33 kann auch aus einem synthetischen oder natürlichen Diamanten bestehen. Diamantenmaterial, z.B. sp3-Kohlenstoff dämpft das IR- und/oder NIR-Licht nicht, ist extrem hart und sehr unempfindlich gegenüber Verschmutzungen gegenüber Lipiden, Proteinen und dgl. Außerdem lässt sich das Diamantenmaterial schnell reinigen.

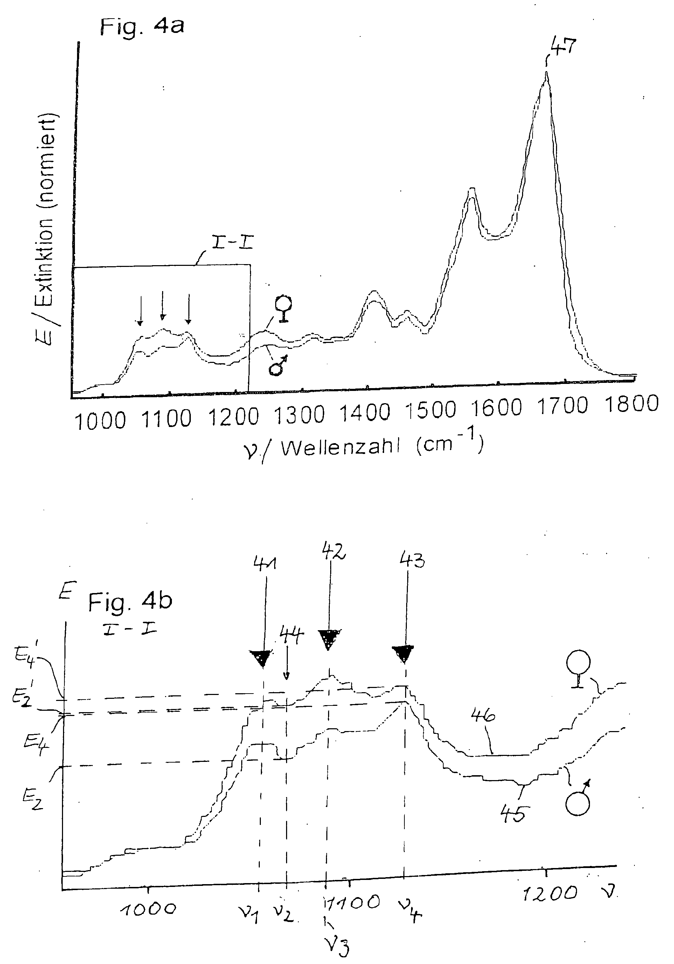

In Fig. 4a und 4b (vergrößerter Ausschnitt) sind FTIR-Spektren 45,46 von Keimscheibenzellen 23 männlicher Vogeleier (45) und weiblicher Vogeleier (46) gezeigt, wobei die Pfeile 41, 42, 43, 44 spektrale Bereiche zur geschlechtsspezifischen Charakterisierung darstellen. Die Untersuchungen zeigen, dass z.B. drei bis vier spektrale Bereiche 41, 42, 43, 44 für die Geschlechtsbestimmung ausreichen, z.B. kann für eine Bestimmung ein Vergleich der Extinktionen in den absoluten Beträgen durchgeführt werden: für das weibliche Geschlecht ist der Extinktionsverlauf zwischen den Wellenzahlen v₁, v₂, v₃, v₄ größer als der Extinktionsverlauf für das männliche Geschlecht zwischen den gleichen Wellenzahlen, so dass in einem Wellenzahlbereich wenigstens ein Extinktionsvergleich stattfinden kann.

Eine andere Möglichkeit der Bestimmung besteht in der Differenzbildung der Extinktion E₄ - E₂ (weiblich) > E₄' - E₂' (männlich), wie in Fig. 4b gezeigt ist.
Die Geschlechtsbestimmung beruht hier z.B. auf den Phosphatschwingungen der DNA- bzw. RNA-Stränge.
Daher kann auch eine Ausführung der Lichtquelle 6 mit optischen Filtern der entsprechenden Wellenzahlen v₁, v₂, v₃, v₄ vorgesehen sein. Es kann anstelle eines teuren Spektrometers 6 zumindest eine oder mehrere Laserdiode/n oder Lichtquelle/n mit einem wellenzahlzugeordneten Filter, insbesondere einem Interferenzfilter bezogen auf die Wellenzahlen v₁, v₂, v₃, v₄ eingesetzt sein, wobei sich die Wellenzahl v aus dem Kehrwert der Wellenlänge A (in Mikrometer) multipliziert mit 10000 ergibt.

Das Verfahren zur Bestimmung des Geschlechts von befruchteten und nicht bebrüteten Vogeleiern 13, wobei ein Vogelei 13 eine feste Eischale 14, ein von der Eischale und weiteren Eihüllen umgebenes Eidotter 2 und eine dem Eidotter 2 zugeordnete Keimscheibe 3 besteht, wobei jeweils zur Aufnahme eines von der Keimscheibe 3 geänderten Spektrums eine Sonde 4 durch ein mikroskopisch kleines Loch 17 der Eischale 14 hindurch in Richtung zur Keimscheibe 3 geführt wird,
weist folgende Schritte auf
- Positionierung der Sonde 4 im Bereich 29 der Keimscheibe 3,
- spektroskopische in-ovo-Charakterisierung der Keimscheibezellen 23,
- Erkennung des Geschlechts durch eine automatische Klassifizierung der rückgeführten Spektren.

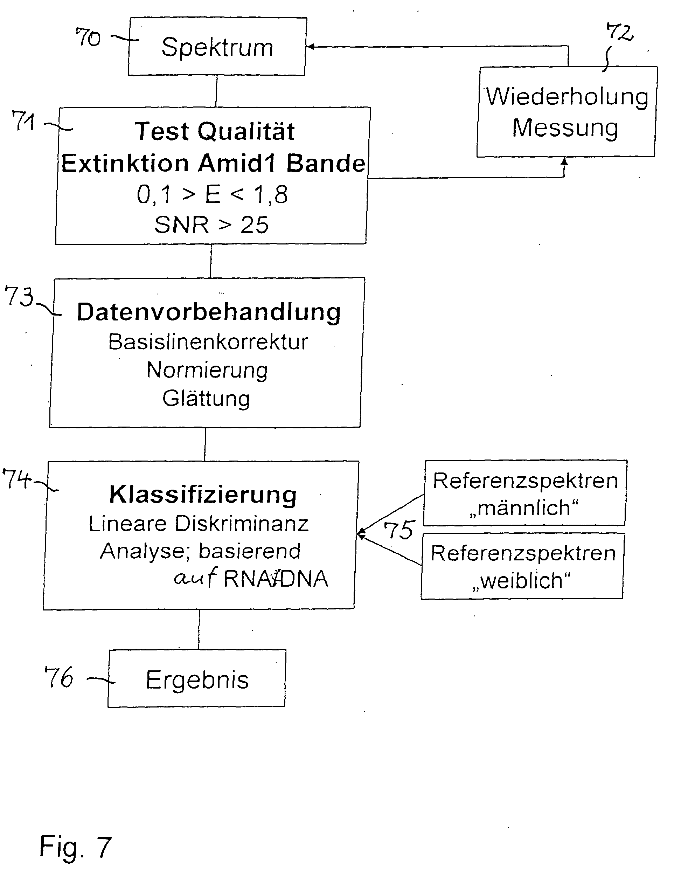

Als Sonde 4 wird ein optischer Kristall eingesetzt, mit dem eine schnelle und rückwirkungsfreie Aufnahme eines Infrarot- und/oder Nahinfrarotspektrums 70, wie in Fig. 7 gezeigt ist, bei Nutzung einer abgeschwächten Totalreflexion 31 innerhalb des optischen Kristalls 4 durch ein außerhalb des optischen Kristalls 4 an einer Ausgangsfläche 27 vorhandenen evaneszentes Feld 21 im Bereich 29 der Keimscheibe 3 durchgeführt wird, wobei eine unterschiedliche Abschwächung/Extinktion infolge einer unterschiedlichen spektralen Absorption von unterschiedlichen geschlechtsspezifischen Keimscheibezellen 23 erfolgt, wobei die Positionierung des optischen Kristalls 4 durch eine permanente automatische Auswertung der kristallinnenseitig totalreflektierten Spektren bis zur Bestimmung der geschlechtsspezifischen Keimscheibezellen 23 begleitet wird, bis das Geschlecht des befruchteten Eis 13 eindeutig angezeigt wird.

Die automatische Positionierung des optischen Kristalls 4 mit der Kristallspitze/Ausgangsffäche 27 wird in Bezug auf die Keimscheibenzellen 3 solange durchgeführt, bis das evaneszente Feld 21 der Totalreflexion 31 an der Kristallspitze/Ausgangsfläche 27 die Keimscheibe 3 erfasst und der optische Kristall 4 seine finale Scheibenzuordnungsposition 28 erreicht hat, bei der das evaneszente Feld 21 in Wechselwirkung mit den Keimscheibenzellen 23 tritt.

Die Scheibenzuordnungsposition 28 des optischen Kristalls 4 kann in alle Raumrichtungen gerichtet möglich sein, um möglichst die Keimscheibe 3 und deren Keimscheibenzellen 23 präzise zu treffen.

Während des Positionierungsvorgangs des optischen Kristalls 4 werden permanent die zurückgeführten totalreflektierten IR- und/oder NIR-Spektren 70 aufgezeichnet und einer Auswertung zugeführt, wobei eine automatische Klassifizierung der rückgeführten Spektren 70 anhand des spektralen Fingerabdruckes in Proteine, Fette und Nukleinsäuren erfolgt.

Bei der geschlechtsspezifischen Absorption des einfallenden IR- und/oder NIR-Lichts werden die Keimscheibenzellen 23 anhand von Absorptionsbanden der Nukleinsäuren (DNA und RNA) im Detektor 32 derart identifiziert, dass das Geschlecht des geprüften Vogeleis 13 bestimmt und angezeigt werden kann.

Die spektroskopische Auswertung wird in der Auswerteeinheit 11 mit der IR- oder NIR-Spektroskopie oder mit einer Fourier-Transform-Infrarot-Spektroskopie durchgeführt, wobei in die Auswertung vorzugsweise ein mathematischer Klassifizierungsalgorithmus einbezogen wird.

In Fig. 7 ist die Funktionsweise mit Einbeziehung der Klassifizierung der gemessenen FTIR-Spektren 70 im Rahmen der Auswertung dargestellt.
Für die Durchführung der spektralen Klassifizierung und deren Ergebnisausgabe wird im Groben ein sechsstufiger Prozess angegeben:
1. Schritt 71 des Qualitätstests der Messung zum Erkennen der Spektren 70 und Eliminieren von unzureichend erkannten Spektren, mit denen keine Auswertung durchgeführt werden kann, wobei geprüft wird, ob die aufgenommenen Spektren 70 folgenden Anforderungen/Kriterien genügen:
   - die Extinktion E der Amid-I Bande 47 als stärkste Absorptionsbande, die in Fig. 4a gezeigt ist, soll im Wesentlichen zwischen 0,1 und 1,8 mit 0,1 > E > 1,8 liegen, wobei über 1,8 hinaus kein linearer Zusammenhang zwischen Konzentration und Extinktion mehr gewährleistet werden kann,
   - das Signal-Rausch-Verhältnis (SNR) der Amid-I Bande hat mindestens eine Größe von 25:1.
2. Schritt 72 der Messwiederholung, falls die beiden Kriterien des Schrittes 71 nicht erreicht werden,
3. Schritt 73 der Datenvorbehandlung mit
   - einer Reduzierung des spektralen Bereiches auf einen Wellenzahlbereich zwischen 1000 und 1800 cm⁻¹, wobei der Wellenbereich den sogenannten Fingerabdruck, die Absorptionen von Proteinen, Lipiden, Nukleinsäuren und weiterer zutreffender Verbindungen, enthält,
   - einer Unterdrückung des Rauschens mittels Savitzky-Golayfilter,
   - einer Korrektur der Basislinie, der linearen Funktion und der Korrektur des Off-sets,
   - einer Normierung der Spektren 70 auf integrale Extinktion durch Flächennormierung.
4. Schritt 74 der spektralen Klassifizierung mit
   der Anwendung einer unterstützten (engl. supervised) Klassifizierung. Als Klassifizierungsverfahren kann die LDA - Lineare Diskriminanz Analyse - eingesetzt werden. Auch andere Verfahren, wie z.B. nichtlineare Verfahren/Methoden oder unterstützende Einrichtungen oder SIMCA, können eingesetzt werden. Die LDA klassifiziert mehrere spektrale Bereiche, also die Extinktionswerte dieser Bereiche. Dabei stützt sich die Klassifizierung hautsächlich auf das Verhältnis der Phosphatschwingungen von DNA und RNA. Durch die Unterschiede im Genom lassen sich männliche Keimscheibenzellen und weibliche Keimscheibenzellen unterscheiden.
5. Schritt 75 der Verifizierung, wobei hierzu ein Referenzspektren-Set mit Referenzspektren "männlich" und Referenzspektren "weiblich" mit bekannter geschlechtlicher Zuordnung benötigt wird. Der Algorithmus vergleicht das Spektrum 70 mit anderen Spektren der Geschlechtsklasse und überprüft die Ähnlichkeit des unbekannten Spektrums 70 mit den bekannten Spektren.
6. Schritt 76 zur Ausgabe der Ergebnisse der Bestimmung des jeweiligen Geschlechts der Vogeleier 13,130.

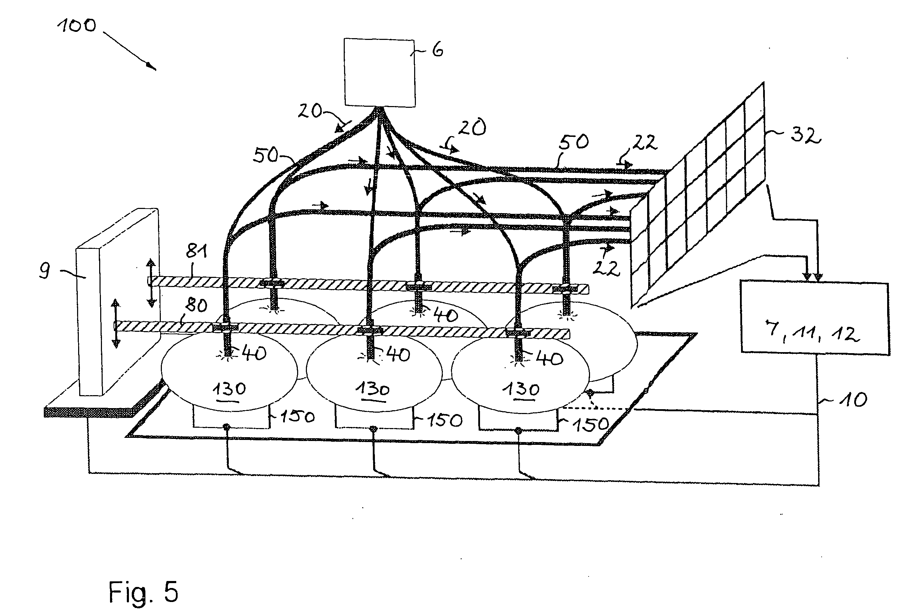

In Fig. 5 ist eine schematische Darstellung einer Vorrichtung 100 zur Bestimmung des Geschlechtes von vielen befruchteten und nicht bebrüteten Vogeleiern 130 mittels einer parallelen Messung der Spektren 70 von vielen Vogeleiern 130 unter Einsatz von optischen Fasern 50, die mit optischen Kristallen 40 verbunden sind, zur Strahlführung gezeigt.
Die Vorrichtung 100 zur Bestimmung des Geschlechtes von befruchteten und nicht bebrüteten Vogeleiern 130 enthält
- mindestens eine Eipositions-Auflage 150 zur Arretierung mindestens eines Eis 130,
- eine Höhenverstelfeinrichtung 9 mit zwei Haltearmen 80,81,
- mehrere als Sonde 40 ausgebildete optische Kristalle, die an den Haltearmen 80,81 befestigt sind,
- eine Steuereinheit 11 zumindest zum Betreiben der eiarretierenden Eipositions-Auflagen 150 und der Höhenverstelleinrichtung 9,
- eine spektrale Lichtquelle 6, die einen IR- und/oder NIR-Lichtstrahl aussendet,
- einen Detektor 32 zur Aufnahme des rückgeführten IR- und/oder NIR-Lichts,
- mehrere optische Elemente 50 für zugehörige geführte Strahlengänge 20 zwischen der spektralen Lichtquelle 6 und den optischen Kristallen 40 sowie für rückgeführte Strahlengänge 22 zwischen den optischen Kristallen 40 und dem Detektor 32 sowie
- eine mit dem Detektor 32 verbundene Auswerteeinheit 7 und eine Anzeigeeinheit 12.

Die Eipositions-Anlage 150 kann in Verbindung mit der Steuereinheit 11 zu einer Einrichtung zur automatischen individuellen Positionierung des betreffenden Eis 130 ausgebildet sein.

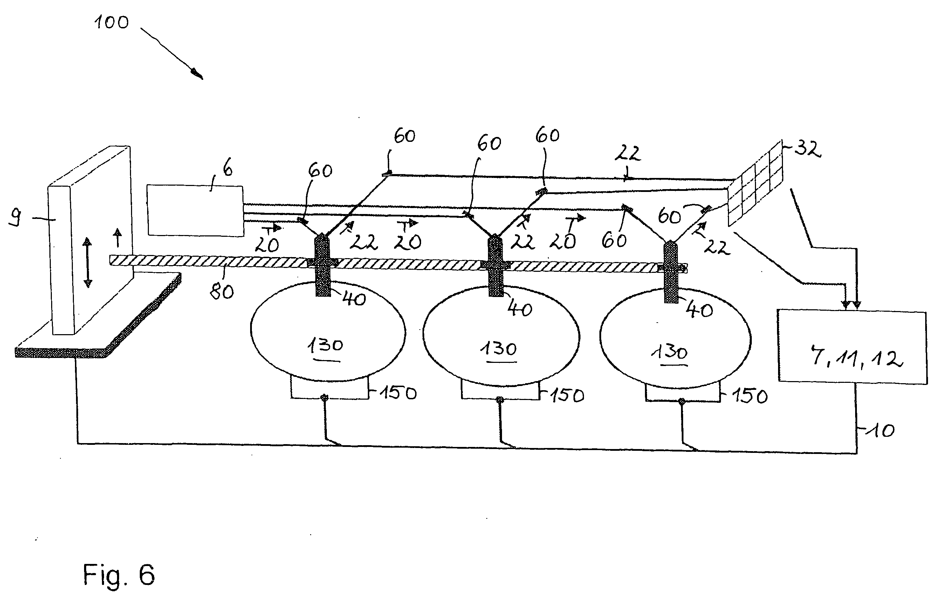

In Fig. 6 ist eine andere schematische Darstellung der Vorrichtung 100 zur Bestimmung des Geschlechtes von befruchteten und nicht bebrüteten Vogeleiern 130 mittels einer parallelen Messung der Spektren 70 von vielen Vogeleiern 130 nach Fig. 5 unter Einsatz von Spiegeln 60 oder Spiegelsystemen zur Strahlführung und einfallswinkelspezifisch angeschliffenen Kristalleingangsflächen 34, wie in Fig. 3 gezeigt, oder Kristallkegeleingangsflächen anstelle der optischen Fasern 50 angegeben.

Gleichzeitig können mit den Vorrichtungen 100 auch mehrere oder viele arraymäßig angeordnete Vogeleier 130 vermessen werden, wobei entweder arraymäßig/feldmäßig mehrere optische Kristalle 40 angeordnet und jeweils mit einer separaten optischen Faser 50 gekoppelt werden oder das totalreflektierte Licht ohne Fasern 50, aber mit Spiegeln 60 direkt auf einen IR- und/oder NIR-empfindlichen Array-Detektor 32 geleitet wird.

### Bezugszeichenliste

- 1: Vorrichtung
- 2: Eidotter
- 3: Keimscheibe
- 4: Optischer Kristall
- 5: Optisches Element
- 6: Lichtquelle für IR- und/oder NIR-Licht
- 7: Auswerteeinheit
- 8: Haltearm
- 9: Höhenverstelleinrichtung
- 10: Versorgungs- und signaltechnische Verbindungsleitungen
- 11: Steuereinheit
- 12: Anzeigeeinheit
- 13: Ei
- 14: Eischale
- 15: Eipositions-Auflage
- 16: Einrichtung zur Feststellung der Lage der Keimscheibe
- 17: Loch
- 18: Goldschicht
- 19: Metallmantel
- 20: geführter Strahlengang
- 21: Evaneszentes Feld
- 22: Rückgeführter Strahlengang
- 23: Keimscheibenzelle
- 24: Dottermembran
- 25: Versorgungs- und signaitechnische Verbindungsleitungen
- 26: Oberfläche
- 27: Untere Ausgangsfläche
- 28: Scheibenzuordnungsposition
- 29: Bereich
- 30: Lage der Keimscheibe
- 31: Totalreflexion
- 32: Detektor
- 33: Kristallspitze
- 34: Kristalfeingangsfläche
- 35: Leitung
- 40: Kristalle
- 41: erster Pfeil für Wellenzahl v₁
- 42: zweiter Pfeil für Wellenzahl v₃
- 43: dritter Pfeil für Wellenzahl v₄
- 44: vierter Pfeil für Wellenzahl v₂
- 45: normierte spektrale männliche Extinktion
- 46: normierte spektrale weibliche Extinktion
- 47: Amid-I- Bande
- 50: Fasern
- 60: Spiegel
- 70: Spektren
- 71: Schritt des Qualitätstests der Messung
- 72: Schritt der Messwiederholung
- 73: Schritt der Datenvorbehandlung
- 74: Schritt der spektralen Klassifizierung
- 75: Schritt der Verifizierung
- 76: Schritt zur Ausgabe der Ergebnisse
- 80: Haltearm
- 81: Haltearm
- 100: Vorrichtung
- 130: Eier
- 150: Eipositions-Auflage
- v₁: Wellenzahl
- v₂: Wellenzahl
- v₃: Wellenzahl
- v₄: Wellenzahl
- E₂: normierte Extinktion bei Wellenzahl v₂
- E₄: normierte Extinktion bei Wellenzahl v₄
- E₂': normierte Extinktion bei Wellenzahl v₂
- E₄': normierte Extinktion bei Wellenzahl v₄

## Patentansprüche

1. Verfahren zur Bestimmung des Geschlechts von befruchteten und nicht bebrüteten Vogeleiern (13,130), wobei ein Ei (13,130) eine feste Eischale (14), und ein von der Eischale und weiteren Eihüllen umgebenes Eidotter (2) und eine dem Eidotter (2) zugeordnete Keimscheibe (3) aufweist, wobei eine Sonde (4,40) zur Messung eines Spektrums (70) durch ein Loch (17) in der Eischale (14) hindurch in Richtung zur Keimscheibe (3) mit Keimscheibenzellen (23) geführt wird,
mit folgenden Schritten
- Positionierung der Sonde (4,40) im Bereich der Keimscheibe (3),
- spektroskopische in-ovo Charakterisierung der Keimscheibenzellen (23),
- Erkennung des Geschlechts durch eine automatische Klassifizierung von Spektren,
wobei als Sonde (4,40) ein optischer Kristall eingesetzt wird, mit dem eine schnelle und rückwirkungsfreie Aufnahme eines Infrarot- und/oder Nahinfrarotspektrums bei Nutzung einer abgeschwächten Totalreflexion (31) innerhalb des optischen Kristalls (4,40) durch ein evaneszentes Feld (21) im Bereich (29) der Keimscheibe (3) durchgeführt wird, wobei die Extinktion infolge einer spektralen Absorption von geschlechtsspezifischen Keimscheibenzellen (23) erfolgt, wobei die Positionierung des optischen Kristalls (4,40) durch eine permanente automatische Auswertung der rückgeführten Spektren (70) bis zur Bestimmung der geschlechlsspezifischen Keimscheibenzellen (23) begleitet wird, bis das Geschlecht des befruchteten Eis (13,130) von einer Auswerteeinheit (7) ausgewertet und von einer Anzeigeeinheit (12) eindeutig angezeigt wird, wobei
die automatische Positionierung des optischen Kristalls (4,40) in Bezug auf die Keimscheibenzellen (23) mit einer Kristallspitze (33) oder Ausgangsfläche (27) solange durchgeführt wird, bis das evaneszente Feld (21) der Totalreflexion (31) an der Kristallspitze (33) oder Ausgangsfläche (27) die Keimscheibe (3) erfasst und der Kristall (4,40) in eine finale Scheibenzuordnungsposition (28) gebracht ist, bei der das evaneszente Feld (21) in Wechselwirkung mit den Keimscheibenzellen (23) tritt, und
wobei während des Positionierungsvorgangs permanent rückgeführte IR-und/oder NIR-Spektren (70) aufgezeichnet und einer Auswertung zugeführt werden, wobei eine automatische Klassifizierung der totalreflektierten Spektren (70) anhand des spektralen Fingerabdruckes in Proteine, Lipiden und Nucleinsäuren erfolgt.

2. Verfahren nach Anspruch 1,
**dadurch gekennzeichnet,**
**dass** das Loch (17) in der Eischale (14) entweder vor der Einbringung des optischen Kristalls (4,40) in die Scheibenzuordnungsposition (28) bereits vorhanden ist oder während des Durchtritts des optischen Kristalls (4,40) durch die Eischale (14) vom Kristall (4,40) selbst eingebracht wird.

3. Verfahren nach Anspruch 1 oder 2,
**dadurch gekennzeichnet,**
**dass** die Scheibenzuordnungsposition (28) des optischen Kristalls (4,40) in alle Raumrichtungen gerichtet möglich ist, um die Keimscheibe (3) präzise zu treffen.

4. Verfahren nach den Ansprüchen 1 bis 3,
**dadurch gekennzeichnet,**
**dass** bei der geschlechtsspezifischen Absorption des einfallenden IR- und/oder NIR-Lichts die Keimscheibenzellen (23) anhand von Absorptionsbanden (41,42,43,44) der Nukleinsäuren (DNA und RNA) derart identifiziert werden, dass das Geschlecht des geprüften Eis (13,130) bestimmt und angezeigt wird.

5. Verfahren nach einem der Ansprüche 1 bis 4,
**dadurch gekennzeichnet,**
**dass** die Auswertung in der Auswerteeinheit (7) mittels der IR- und/oder NIR-Spektroskopie oder der Fourier-Transform-Infrarot-Spektroskopie durchgeführt wird.

6. Verfahren nach einem der Ansprüche 1 bis 5,
**dadurch gekennzeichnet,**
**dass** gleichzeitig mehrere Eier (130) in einer Feldanordnung und voneinander beabstandet vermessen werden, wobei entweder feldartig voneinander beabstandet mehrere optische Kristalle (40) angeordnet werden, die jeweils mit einer separaten optischen Faser (50) gekoppelt sind oder wobei das Licht mittels Spiegel (60) oder Spiegelsystemen zur Strahlablenkung von der spektralen Lichtquelle (6) aus und nach geschlechtsspezifischer Absorption auf einen IR- und/oder NIR-empfindlichen Detektor (32) mit feldartig voneinander beabstandeten Detektorelementen oder Pixeln geleitet wird.

7. Vorrichtung (1, 100) zur Bestimmung des Geschlechtes von befruchteten und nicht bebrüteten Vogeleiern (13,130), umfassend eine durch ein Loch (17) einer Eischale (14) eines Eis in Richtung zur Keimscheibe (3) mit Keimscheibenzellen (23) führbare Sonde (4,40) zur Messung eines Spektrums nach dem Verfahren gemäß einem der Ansprüche 1 bis 6, enthaltend
- mindestens eine Eipositions-Auflage (15, 150) zur Arretierung mindestens eines Eis (13,130),
- mindestens eine Höhenverstelleinrichtung (9) mit mindestens einem Haltearm (8,80,81),
- mindestens einen als Sonde (4,40) ausgebildeten optischen Kristall, der an dem Haltearm (8,80,81) befestigt ist,
- mindestens eine Steuereinheit (11) zumindest zum Betreiben der eiarretierenden Eipositions-Auflage (15,150) und der Höhenverstelleinrichtung (9),
- mindestens eine spektrale Lichtquelle (6), die einen IR- und/oder NIR-Lichtstrahl aussendet,
- mindestens einen Detektor (32) zur Aufnahme des rückgeführten IR- und/oder NIR-Lichts,
- mindestens ein optisches Element (5,50,60) für einen geführten Strahlengang (20) zwischen der spektralen Lichtquelle (6) und dem optischen Kristall (4,40) sowie für einen rückgeführten Strahlengang (22) zwischen dem optischen Kristall (4,40) und dem Detektor (32) sowie
- eine mit dem Detektor (32) verbundene Auswerteeinheit (7) und eine Anzeigeeinheit (12),
wobei mit der Höhenverstelleinrichtung (9) die Höhe des Haltearms (8) und somit des optischen Kristalls (4,40) in Bezug auf die Lage (30) der Keimscheibe (3) einstellbar ist und der optische Kristall (4,40) im Bereich (29) der Keimscheibe (3) in einer Scheibenzuordnungsposition (28) positionierbar ist, in der über den optischen Kristall (4,40) ein sich bei Totalreflexion (31) an der zur Keimscheibe (3) gerichteten Ausgangsfläche (27) ausbildendes evaneszentes Feld (21) auf die Keimscheibe (3) übergreift und die darin befindlichen Keimscheibenzellen (23) wechselwirkend mit dem evaneszenten Feld (21) eine geschlechtsspezifische Absorption des Lichts aus dem eingeführten Strahlengang (20) vornehmen, wobei das an der Ausgangsfläche (27) totalreflektierte Licht über den rückgeführten Strahlengang (22) innerhalb des Kristalls (4,40) und schließlich über das optische Element (5,50,60) zur Registrierung zum Detektor (32) geführt wird, von dem aus die registrierten spektralen Signale (E₂,E₄,E₂',E₄';41,42,43,44) zur Auswertung und Anzeige des Geschlechts übermittelt werden,
wobei eine Einrichtung (16) zur Feststellung der Lage (30) der Keimscheibe (3), die Höhenverstelleinrichtung (9) und die Eipositions-Auflage (15,150) in der Steuereinheit (11) mittels programmtechnischer Mittel zu einer Koordinierung der Lage (30) der Keimscheibe (3) und der Scheibenzuordnungsposition (28) des optischen Kristalls (4,40) verbunden sind und zur Festlegung einer präzisen Scheibenzuordnungsposition (28) des optischen Kristalls (4,40) im Bereich (29) der Keimscheibe (3) ausgebildet sind.

8. Vorrichtung nach Anspruch 7,
**dadurch gekennzeichnet,**
**dass** mit der Einrichtung (16) zur Feststellung der Lage (30) der Keimscheibe (3) innerhalb des Eis (13,130) über den Haltearm (8,80,81), der höhenverstellbar und verschwenkbar ausgebildet ist, die Scheibenzuordnungsposition (28) des optischen Kristalls (4,40) im Bereich (29) der Keimscheibe (3) einstellbar ist.

9. Vorrichtung nach Anspruch 7,
**dadurch gekennzeichnet,**
**dass** die Einrichtung (16) zur Feststellung der Lage (30) der Keimscheibe (3) über mindestens eine versorgungs- und signaltechnische Leitung (25) mit der Steuereinheit (11) verbunden ist.

10. Vorrichtung nach Anspruch 7,
**dadurch gekennzeichnet,**
**dass** das optische Element (5,50) eine flexible optische Faser ist und der optische Kristall (4,40) mit der flexiblen optischen Faser (5,50) verbunden ist, wobei der Lichtstrahl sowohl von der spektralen Lichtquelle (6) zum optischen Kristall (4,40) als auch vom optischen Kristall (4,40) zum Detektor (32) durch die Faser (5,50) geleitet wird.

11. Vorrichtung nach Anspruch 7,
**dadurch gekennzeichnet,**
**dass** der optische Kristall (4,40) mit verschieden ausgebildeten Kristallendflächen oder Kristallenden, mit einer ebenen, einer runden oder ovalen Ausgangsfläche (27) oder aber auch mit einer spitzen Kristallspitze (33) versehen ist.

12. Vorrichtung nach den Ansprüchen 7 bis 11,
**dadurch gekennzeichnet,**
**dass** der optische Kristall (4,40) zylindrisch ausgebildet ist und sich in einem Metallmantel (19) gehaltert befindet, wobei der Metallmantel (19) mit dem Haltearm (8,80,81) in Verbindung steht.

13. Vorrichtung nach Anspruch 12,
**dadurch gekennzeichnet,**
**dass** zwischen dem Metallmantel (19) und der Oberfläche (26) des optischen Kristalls (4,40) zur Verbesserung der Reflexion an der Oberfläche (26) eine Goldschicht (18) angebracht ist.

14. Vorrichtung nach Anspruch 12,
**dadurch gekennzeichnet,**
**dass** der optische Kristall (4,40) nadelförmig im Bereich der unteren Ausgangsfläche (27) ausgebildet ist.

15. Vorrichtung nach Anspruch 12,
**dadurch gekennzeichnet,**
**dass** der optische Kristall (4,40) aus IR-transparentem und zugleich hartem Material aus Germanium, Silizium, Kalziumfluorid (CaF₂), Bariumfluorid (BaF₂), Zinkselenid (ZnSe) oder Zinksulfid (ZnS) besteht.

16. Vorrichtung nach Anspruch 7,
**dadurch gekennzeichnet,**
**dass** der optische Kristall (4,40) für Messungen mit NIR-Licht aus Glas oder Saphir besteht.

17. Vorrichtung nach Anspruch 7 oder 12,
**dadurch gekennzeichnet,**
**dass** der optische Kristall (4,40) aus einem Komposit besteht, wobei bei der Nadelförmigkeit des Kristalls (4,40) die Kristallspitze (33) im Bereich der Ausgangsfläche (27) aus Diamant besteht.

18. Vorrichtung nach einem der Ansprüche 7 oder 8 bis 17,
**dadurch gekennzeichnet,**
**dass** als IR-und/oder NIR-sensitiver Detektor (32) ein pixellierter Detektor eingesetzt ist, um gleichzeitig und parallel rückgeführte Spektren (70) von vielen zu prüfenden Eiern (13,130) aus den zugeordneten optischen Kristallen (4,40) pixelzugeordnet zu erfassen.

## Claims

1. Method to determine the sex of fertilised and non-incubated bird's eggs (13,130), where an egg (13,130) has a firm egg shell (14) and an egg yolk (2) surrounded by the egg shell and other integuments as well as a blastodisc (3) related to the egg yolk (2), whereby a sensor (4,40) for measuring a spectrum (70) is inserted through a hole (17) in the egg shell (14) towards the blastodisc (3) with blastodisc cells (23),
with the following steps
- positioning of the sensor (4,40) in the vicinity of the blastodisc (3),
- spectroscopic in-ovo characterisation of the blastodisc cells (23),
- determination of the sex through an automatic classification of spectrums, whereby an optical crystal is used as the sensor (4,40), with which a fast and non-reactive absorption of an infrared and/or near-infrared spectrum is performed with utilisation of a attenuated total reflexion (31) within the optical crystal (4,40) due to an evanescent field (21) in the area (29) of the blastodisc (3), wherein the extinction results following a spectral absorption of sex specific blastodisc cells (23), wherein a permanent automatic analysis of the returned spectrums (70) is performed at the same time as positioning of the optical crystal (4,40) until the sex specific blastodisc cells (23) are determined until the sex of the fertilised egg (13,130) is analysed by an analysis unit (7) and clearly indicated by a display unit (12), whereby
automatic positioning of the optical crystal (4,40) in relation to the blastodisc cells (23) is performed with a crystal tip (33) or a working face (27) until the evanescent field (21) of the total reflexion (31) captures the blastodisc (3) at the crystal tip (33) or the working face (27) and the crystal (4,40) is brought into a final disc allocation position (28) where the evanescent field (21) starts to interact with the blastodisc cells (23), and
wherein IR and/or NIR spectrums (70) continuously returned during the positioning process are recorded and subjected to analysis, wherein the totally reflected spectrums (70) are automatically classified using the spectral fingerprint in proteins, lipids and nucleic acids.

2. Method according to Claim 1,
**characterized in that**
the hole (17) in the egg shell (14) is either already present before inserting the optical crystal (4,40) into the disc allocation position (28) or is created by the crystal (4,40) itself when the optical crystal (4,40) penetrates the egg shell (14).

3. Method according to Claim 1 or 2,
**characterized in that**
the disc allocation position (28) of the optical crystal (4,40) is possible with all spatial orientations to hit the blastodisc (3) accurately.

4. Method according to Claims 1 to 3,
**characterized in that**
during sex-specific absorption of the incoming IR and/or NIR light, the blastodisc cells (23 are identified using absorption bands (41,42,43,44) of the nucleic acids (DNA and RNA) in such a way that the sex of the tested egg (13,130) is determined and displayed.

5. Method according to one of Claims 1 to 4,
**characterized in that**
the analysis in the analysis unit (7) is done using IR and/or NIR spectroscopy or with Fourier transform infrared spectroscopy.

6. Method according to one of Claims 1 to 5,
**characterized in that**
several eggs (130) are measured in a field arrangement and with a distance between them, wherein either several optical crystals (40) are positioned in a field with a distance between them, which are connected with a separate optical fibre (50), or using a mirror (60) or mirror system the light is directed from the spectral light source (6) and, after sex-specific absorption, is directed to an IR and/or NIR-sensitive detector (32) in a field with a distance between the detector elements or pixels.

7. Apparatus (1,100) to determine the sex of fertilised and non-incubated eggs (13,130) comprising a sensor (4,40) that can be guided through a hole (17) of an egg shell (14) of an egg towards the blastodisc (3) with blastodisc cells (23), to measure a spectrum using the procedure according to one of the Claims 1 to 6, including
- at least on egg positioning plate (15,150) to adjust at least one egg (13,130),
- at least one height adjustment device (9) with at least one holding arm (8,80,81),
- with at least one optical crystal prepared as sensor (4,40), which is mounted to the holding arm (8,80,81),
- at least on control unit (11) at least to operate the egg-adjusting egg positioning plate (15,150) and the height adjustment device (9),
- at least one spectral light source (6) emitting an IR and/or NIR light beam,
- at least one detector (32) to absorb the returned IR and/or NIR light,
- at least one optical element (5,50,60) for a guided optical path (20) between the spectral light source (6) and the optical crystal (4,40) and for a returned optical path (22) between the optical crystal (4,40) and the detector (32) as well as
- one analysis unit (7) connected to the detector (32) and a display unit (12),
wherein the height of the holding arm (8) and therefore of the optical crystal (4,40) can be adjusted with respect to the position (30) of the blastodisc (3) with the height adjustment device (9), and the optical crystal (4,40) can be positioned in the area (29) of the blastodisc (3) in a disc allocation position (28), where, in the event of total reflexion (31), an evanescent field (21) forming over the optical crystal (4,40) at the initial surface (27) that is directed to the blastodisc (3) overlaps the blastodisc (3) and the blastodisc cells (23) therein absorb the light from the inserted optical path (20) in a sex-specific manner in interaction with the evanescent field (21), wherein light totally reflected at the initial surface (27) is then guided via the returning optical path (22) within the crystal (4,40) and the optical element (5,50,60) to the detector (32) for registration, from where the registered spectral signals (E₂,E₄,E₂',E₄';41,42.43,44) are transmitted for analysis and display of the sex,wherein an apparatus (16) to determine the position (30) of the blastodisc (3), the height adjustment device (9) and the egg positioning plate (15,150) are connected in the control unit (11) using programmable means for coordination of the position (30) of the blastodisc (3) and the disc allocation position (28) of the optical crystal (4,40) and are designed to determine an accurate disc allocation position (28) of the optical crystal (4,40) in the area (29) of the blastodisc (3).

8. Apparatus according to Claim 7,
**characterized in that**
the disc allocation position (28) of the optical crystal (4,40) can be adjusted in the area (29) of the blastodisc (3) with the apparatus (16) used to determine the position (30) of the blastodisc (3) within the egg (13,130) using the holding arm (8,80,81), which has an adjustable height and can be tilted.

9. Apparatus according to Claim 7,
**characterized in that**
the apparatus (16) for determination of the position (30) of the blastodisc (3) has at least one supply and signal line (25) to which the control unit (11) is connected.

10. Apparatus according to Claim 7,
**characterized in that**
the optical element (5,50) is a flexible optical fibre and the optical crystal (4,40) is connected to the flexible optical fibre (5,50), wherein the light beam is guided through the fibre (5,50) from the spectral light source (6) to the optical crystal (4,40) and also from the optical crystal (4,40) to the detector 32).

11. Apparatus according to Claim 7,
**characterized in that**
the optical crystal (4,40) has different shaped crystal end faces or crystal ends with one even, one round or oval working face (27) or even with a pointed crystal tip (33).

12. Apparatus according to Claims 7 and 11,
**characterized in that**
the optical crystal (4,40) is shaped as a cylinder and held inside a metal sleeve (19), wherein the metal sleeve (19) is connected to the holding arm (8,80,81).

13. Apparatus according to Claim 12,
**characterized in that**
a gold layer (18) is inserted between the metal sleeve (19) and the surface (26) of the optical crystal (4,40) to improve the reflexion at the surface (26).

14. Apparatus according to Claim 12,
**Characterized in that**
the optical crystal (4,40) is shaped in the form of a needle near the lower working face (27).

15. Apparatus according to Claim 12,
**characterized in that**
the optical crystal (4,40) is made of IR-transparent and hard material consisting of germanium, silicon, calcium fluoride (CaF₂), barium fluoride (BaF₂), zinc selenid (ZnSe) or zink sulfide (ZnS).

16. Apparatus according to Claim 7,
**characterized in that**
the optical crystal (4,40) is made of glass or sapphire for measurements with NIR light.

17. Apparatus according to Claim 7 or 12,
**Characterized in that**
the optical crystal (4,40) consists of a composite, whereby the crystal tip (33) near the working face (27) is made of diamond due to the needle-shape of the crystal (4,40).

18. Apparatus according to one of the Claims 7 or 8 to 17,
**characterized in that**
a pixellated detector is used as IR and/or NIR-sensitive detector (32) to detect spectrums (70) that are returned from the allocated optical crystals (4,40) at the same time and in parallel from many eggs (13,130) to be tested.

## Revendications

1. Procédé permettant de déterminer le sexe d'oeufs d'oiseaux fécondés et non incubés (13,130), un oeuf (13,130) comprenant une coquille d'oeuf solide (14) un jaune d'oeuf (2) entouré par la coquille d'oeuf et d'autres membranes d'oeuf et un disque germinatif (3) associé au jaune d'oeuf (2), une sonde (4,40) de mesure d'un spectre (70) étant introduite par un trou (17) au travers de la coquille d'oeuf (14) dans la direction du disque germinatif (3) comprenant des cellules de disque germinatif (23),
procédé comprenant les étapes suivantes consistant à :
- positionner la sonde (4,40) dans la zone du disque germinatif (3),
- caractériser in-ovo par spectroscope les cellules du disque germinatif (23),
- identifier le sexe par une classification automatique des spectres,
un cristal optique, utilisé en tant que sonde (4,40), effectuant un enregistrement rapide et sans effet rétroactif d'un spectre infrarouge et/ou dans le proche infrarouge, en utilisant une réflexion totale atténuée (31) à l'intérieur du cristal optique (4,40) par un champ évanescent (21) dans la zone (29) du disque germinatif (3), l'extinction s'effectuant par absorption spectrale par des cellules du disque germinatif (23) spécifiques au sexe, le positionnement du cristal optique (4,40) étant accompagné d'une évaluation automatique permanente des spectres renvoyés (70) jusqu'à la détermination des cellules du disque germinatif (23) spécifiques au sexe, ce jusqu'à ce que le sexe de l'oeuf fécondé (13,130) soit évalué par une unité d'évaluation (7) et indiqué de façon incontestable par une unité d'affichage (12),
le positionnement automatique du cristal optique (4,40) par rapport aux cellules du disque germinatif (23) étant effectué avec une pointe de cristal (33) ou une surface de sortie (27) jusqu'à ce que le champ évanescent (21) de la réflexion totale (31) sur la pointe de cristal (33) ou la surface de sortie (27) saisisse le disque germinatif (3) et que le cristal (4,40) soit placé dans une position d'association au disque (28) finale dans laquelle le champ évanescent (21) interagit avec les cellules du disque germinatif (23) et
pendant le procédé de positionnement des spectres IR et/ou NIR (70) à renvoi permanent étant enregistrés et envoyées à une unité d'évaluation, une classification automatique des spectres (70) à réflexion totale étant effectuée sur le fondement de l'empreinte digitale spectrale en protéines, lipides et acides nucléiques.

2. Procédé selon la revendication 1
**caractérisé en ce que**
le trou (17) dans la coquille d'oeuf (14) existe déjà avant la mise en place du cristal optique (4,40) dans la position d'association au disque (28) ou est formé lors de la pénétration du cristal optique (4,40) dans la coquille d'oeuf (14) par ce cristal (4,40) lui-même.

3. Procédé selon la revendication 1 ou 2
**caractérisé en ce que**
la position d'association au disque (28) du cristal optique (4,40) peut être orientée dans toutes les directions spatiales pour atteindre avec précision le disque germinatif (3).

4. Procédé selon les revendications 1 à 3
**caractérisé en ce que**
lors de l'absorption spécifique au sexe de la lumière IR et/ou NIR incidente, les cellules du disque germinatif (23) sont identifiées à l'aide de bandes d'absorption (41,42,43,44) des acides nucléiques (ADN et ARN) de façon que le sexe de l'oeuf testé (13,130) soit déterminé et indiqué.

5. Procédé selon l'une des revendications de 1 à 4
**caractérisé en ce que**
l'évaluation dans l'unité d'évaluation (7) est effectuée par spectroscopie IR et/ou NIR ou par spectroscopie infrarouge à transformée de Fourier.

6. Procédé selon l'une des revendications de 1 à 5
**caractérisé en ce que**
plusieurs oeufs (130) positionnés selon un réseau et situés à distance les unes des autres sont mesurés simultanément, plusieurs cristaux optiques (40) respectivement couplés avec une fibre optique séparée (50) étant positionnés en réseau à distance les uns des autres de l'autre ou la lumière étant dirigée par un miroir (60) ou des systèmes de miroirs permettant de dévier le faisceau provenant de la source lumineuse spectrale (6) et après absorption spécifique au sexe dirigée sur un détecteur sensible aux IR et/ou NIR (32) ayant des éléments de détecteur ou des pixels positionnés selon un réseau et situés à distance les uns des autres.

7. Dispositif (1,100) permettant de déterminer le sexe d'oeufs d'oiseaux fécondés et non incubés (13,130), comprenant une sonde (4,40) pouvant être introduite par un trou (17) d'une coquille (14) d'un oeuf dans la direction du disque germinatif (3) comprenant des cellules de disque germinatif (23) pour permettre la mesure d'un spectre par la mise en oeuvre du procédé selon l'une des revendications 1 à 6, comprenant
- au moins un support de position d'oeuf (15,150) pour bloquer au moins un oeuf (13,130),
- au moins un dispositif de réglage de hauteur (9) comprenant au moins un bras de maintien (8,80,81),
- au moins un cristal optique réalisé en tant que sonde (4,40)à qui est fixé au bras de maintien (8,80,81),
- au moins une unité de commande (11) au moins pour actionner le support de position d'oeuf (15,150) permettant de bloquer l'oeuf et le dispositif de réglage de hauteur (9),
- au moins une source de lumière spectrale (6) qui émet un faisceau lumineux IR et/ou NIR,
- au moins un détecteur (32) pour recevoir la lumière IR et/ou NIR renvoyée,
- au moins un élément optique (5,50,60) permettant de guider le rayonnement (20) entre la source de lumière spectrale (6) et le cristal optique (4,40) ainsi que le rayonnement renvoyé (22) entre le cristal optique (4,40) et le détecteur (32),
- ainsi qu'une unité d'évaluation (7) associée au détecteur (32) et une unité d'affichage (12),
dispositif dans lequel
le dispositif de réglage de hauteur (9) permet de régler la hauteur du bras de maintien (8) et donc du cristal optique (4,40) par rapport à la position (30) du disque germinatif (3) et de positionner le cristal optique (4,40) dans la zone (29) du disque germinatif (3) dans une position d'association au disque (28) dans laquelle un champ évanescent (21) se formant par l'intermédiaire du cristal optique (4,40) par réflexion totale (31) à la surface de sortie (27) dirigée vers le disque germinatif (3) se propage au disque germinatif (3) et les cellules du disque germinatif (23) qui y sont situées procèdent à une absorption de la lumière provenant du rayonnement entrant spécifique au sexe en interagissant avec le champ évanescent (21), la lumière totalement réfléchie à la surface de sortie (27) est guidée par l'intermédiaire du rayonnement renvoyé (22) à la partie interne du cristal (4,40) et enfin par l'intermédiaire de l'élément optique (5,50,60), pour permettre son enregistrement, vers le détecteur (32) qui transmet les signaux spectraux enregistrés (E₂,E₄,E₂',E₄';41,42,43,44) pour permettre leur évaluation et l'affichage du sexe,
un dispositif (16) permettant de déterminer la position (30) du disque germinatif (3), le dispositif de réglage de hauteur (9) et le support de position d'oeuf (15,150) sont reliés dans l'unité de commande (11) par des moyens de programmation pour permettre une coordination de la position (30) du disque germinatif (3) et de la position d'association au disque (28) du cristal optique (4,40) et, réalisés pour permettre de déterminer une position d'association au disque (28) précise du cristal optique (4,40) dans la zone (29) du disque germinatif (3) étant formés.

8. Dispositif selon la revendication 7
**caractérisé en ce que**
le dispositif (16) permettant de déterminer la position (30) du disque germinatif (3) à la partie interne de l'oeuf (13,130) permet, par l'intermédiaire du bras de maintien (8,80,81) qui est réglable en hauteur et pivotant, de régler la position d'association au disque (28) du cristal optique (4,40) dans la zone (29) du disque germinatif (3).

9. Dispositif selon la revendication 7
**caractérisé en ce que**
le dispositif (16) permettant de déterminer la position (30) du disque germinatif (3) est relié à l'unité de commande (11) par l'intermédiaire d'au moins une conduite d'alimentation et de transfert de signaux (25).

10. Dispositif selon la revendication 7
**caractérisé en ce que**
l'élément optique (5,50) est une fibre optique flexible et le cristal optique (4,40) est relié à l'élément optique flexible (5,50), le faisceau lumineux étant guidé par les fibre (5,50) de la source de lumière spectrale (6) au cristal optique (4,40) ainsi que du cristal optique (4,40) au détecteur (32).

11. Dispositif selon la revendication 7
**caractérisé en ce que**
le cristal optique (4,40) est muni de surfaces d'extrémité ou d'extrémités différentes ayant une surface de sortie (27) plane, arrondie ou ovale ou toutefois aussi une pointe de cristal (33) pointue.

12. Dispositif selon les revendications 7 à 11
**caractérisé en ce que**
le cristal optique (4,40) est réalisé sous forme de cylindre et est maintenu dans une gaine métallique (19), cette gaine métallique (19) étant reliée au bras de maintien (8,80,81).

13. Dispositif selon la revendication 12
**caractérisé en ce qu'**
une couche d'or (18) est appliquée entre la gaine métallique (19) et la surface (26) du cristal optique (4,40) pour améliorer la réflexion à la surface (26).

14. Dispositif selon la revendication 12
**caractérisé en ce que**
le cristal optique (4,40) est en forme d'aiguille dans la zone de la surface de sortie inférieure (27).

15. Dispositif selon la revendication 12
**caractérisé en ce que**
le cristal optique (4,40) se réalisé en un matériau transparent aux IR et en même temps dur, à base de germanium, silicium, fluorure de calcium (CaF₂), fluorure de baryum (BaF₂), séléniure de zinc (ZnSe) ou sulfure de zinc (ZnS).

16. Dispositif selon la revendication 7
**caractérisé en ce que**
le cristal optique (4,40) est réalisé en verre ou en saphir pour permettre des mesures avec de la lumière NIR.

17. Dispositif selon la revendication 7 ou 12
**caractérisé en ce que**
le cristal optique (4,40) est réalisé en un matériau composite, et s'il est en forme d'aiguille, la pointe (33) de ce cristal optique (4,40) est constituée de diamant dans la zone de la surface de sortie (27).

18. Dispositif selon l'une des revendications 7 ou 8 à 17
**caractérisé en ce que**
comme détecteur (32) sensible aux IR et/ou NIR, on utilise un détecteur pixellisé pour permettre de détecter de façon associée aux pixels à partir des cristaux (4,40) associés des spectres (70) renvoyés simultanément et parallèlement de nombreux oeufs à tester (13,130).
